# EUROPÄISCHE PATENTSCHRIFT

(11) **EP 1 054 963 B1**
(45) Veröffentlichungstag und Bekanntmachung des Hinweises auf die Patenterteilung: **29.08.2007**
(21) Anmeldenummer: 99907550.0
(22) Anmeldetag: 12.02.1999
(51) Int. Cl.: C12N 15/12, C07K 14/705, G01N 33/50, C12Q 1/68, A61K 38/17, C07K 16/28, C12N 15/11, A61K 48/00

(54) **SEQUENZEN EINES IH-IONENKANALS UND DEREN VERWENDUNG**
SEQUENCES OF AN IH IONIC CHANNEL AND THE USE THEREOF
SEQUENCES D'UN CANAL IONIQUE IH ET LEUR UTILISATION

(30) Priorität: 17.02.1998 DE 19806581
(43) Veröffentlichungstag der Anmeldung: 29.11.2000
(60) Teilanmeldung: 07003897.1
(73) Patentinhaber: Forschungszentrum Jülich GmbH, 52425 Jülich (DE)
(72) Erfinder: BAUMANN, Arnd, D-51528 Jülich (DE); BÖNIGK, Wolfgang, D-52428 Jülich (DE); GAUSS, Renate, D-95444 Bayreuth (DE); SCHOLTEN, Alexander Carl von Ossietzky Universität Oldenburg Institut für Biologie u. Umweltwissenschaften Biochemie, D-26129 Oldenburg (DE); SEIFERT, Reinhard, D-52428 Jülich (DE); KAUPP, Benjamin, D-52062 Aachen (DE)
(74) Vertreter: Grünecker, Kinkeldey, Stockmair & Schwanhäusser Anwaltssozietät
(86) Internationale Anmeldenummer: PCT/EP1999/000942
(87) Internationale Veröffentlichungsnummer: WO 1999/042574

(56) Entgegenhaltungen:
- WO-A-99/32615
- WO-A-99/37660
- SANTORO B ET AL: "Interacitvie cloning with the SH3 domain of N-src identifies a new brain specific ion channel protein, with homology to Eag and cyclic nucleotide-gated channels" PROCEEDINGS OF THE NATIONAL ACADEMY OF SCIENCES OF USA., Bd. 94, Dezember 1997 (1997-12), Seiten 14815-14820, XP002109143 NATIONAL ACADEMY OF SCIENCE. WASHINGTON., US ISSN: 0027-8424
- SANTORO B ET AL: "Identification of a gene encoding a hyperpolarization-activated pacemaker channel of brain" CELL, Bd. 93, 29. Mai 1998 (1998-05-29), Seiten 717-729, XP002097362 ISSN: 0092-8674
- GAUSS R ET AL: "Molecular identification of a hyperpolarization -activated channel in sea urchin sperm" NATURE, Bd. 393, 11. Juni 1998 (1998-06-11), Seiten 583-587, XP002109144 LONDON GB
- LUDWIG A ET AL: "A family of hyperpolarization-activated mammalian cation channels" NATURE, Bd. 393, 11. Juni 1998 (1998-06-11), Seiten 587-591, XP002109145 LONDON GB
- HILLIER L ET AL: "Homo sapiens cDNA clone similar to DmCNGC protein-fruit fly" EMHUM DATABASE ENTRY HSN72770, ACCESSION NUMBER N72770, XP002109146

## Beschreibung

Die vorliegende Erfindung betrifft eine Nukleinsäure vorzugsweise eine DNA, die eine Sequenz eines menschliches Iₕ-Ionenkanals umfaßt. Ferner betrifft die vorliegende Erfindung ein mRNA-Molekül, das die entsprechende Sequenz umfaßt. Die Erfindung betrifft außerdem ein Polypeptid bzw. Protein, das die entsprechende abgeleitete Aminosäuresequenz umfaßt. Die Ausführungsformen der Erfindung sind in den Ansprüchen 1-18 dargelegt.

Die Erfindung betrifft dabei auch die Verwendung einer oder mehrerer der oben genannten Sequenzen für ein Screeningverfahren und/oder zur Verwendung in der Diagnose sowie die dafür notwendigen Kits.

Schließlich betrifft die Erfindung die 2. medizinische Verwendung einer oder mehrerer der obenstehenden Sequenzen für die Behandlung und/oder Prophylaxe von Herz/Kreislaufstörungen sowie Schlafstörungen.

Die große Vielfalt der Funktionen des Nervensystems wird wesentlich bestimmt durch fein abgestimmte Wechselwirkungen zwischen den intrinsischen Eigenschaft der Neuronen und den synaptischen Verbindungen. Die den Neuronen und Synapsen eigenen elektrophysiologischen Eigenschaften wiederum werden bestimmt von der Lokalisierung und Dichte der spannungs- und liganden-gesteuerten Ionenkanäle, die den Fluß von Ionenströmen über die neuronale Plasmamembran regulieren und die durch eine große Vielzahl von Transmittersubstanzen und intrazellulären Boten-Systemen gesteuert werden (Hille, 1992)

Im Hinblick auf die spezifische Aktivität, die von den neuronalen Elementen erwartet wird, ist es nicht erstaunlich, daß Neuronen ein großes Repertoire an Ionenkanälen besitzen, einschließlich der klassischen Kanäle, die spannungsabhängige Natrium(Na⁺)- und Kalium(K⁺)- Ströme während eines Aktionspotentials erzeugen (Hodgkin und Huxley, 1952) wie auch eine Anzahl ungewöhnlicher Ionenleitwerte (Llinás, 1988).

Ein ursprünglich von Ito und Mitarbeitern (Araki et al., 1962; Ito und Oshima, 1965) in Motoneuronen von Katzen entdeckter, ungewöhnlicher intrinsischer Mechanismus erwies sich als langsame Relaxation der Potentialveränderung, die durch hyperpolarisierenden Strom induziert wird, was zu einem nicht ohmischen Verhalten der Strom/Spannungs(IN)-Beziehung in hyperpolarisierender Richtung führt. Der zugrunde liegende, zeitabhängige Membranstrom wurde zunächst in Photorezeptoren der Stäbchen als Cäsium(Cs⁺)-empfindlicher, nach innen gerichteter Strom charakterisiert, der durch Hyperpolarisierung ausgelöst wird und die Membran depolarisieren kann. Dies führt zu der typischen Abfolge einer anfänglichen transienten Hyperpolarisierung durch Belichtung, gefolgt von einer langsamen Depolarisation (Attwell und Wilson, 1980; Bader et al., 1982; Bader et al., 1979; Fain et al., 1978).

Der Strom in den Photorezeptoren wurde als Iₕ bezeichnet, da er durch eine Hyperpolarisierung aktiviert wird. Ungefähr zur selben Zeit wurde ein ähnlicher Ionenstrom im Herz, in den Schrittmacherzellen des Sinusknoten, entdeckt sowie in den Purkinje-Fasern des Säugerherzens (Brown und DiFrancesco, 1980; Brown et al., 1979; DiFrancesco, 1981a; DiFrancesco, 1981b; Yanagihara und Irisawa, 1980) und es wurde deutlich, daß der langsame, nach innen gerichtete Strom von Natrium- und Kaliumionen getragen wird. Dieser Strom wurde als "funny"-Strom bezeichnet (l_{f}), um sein ungewöhnliches Verhalten zu unterstreichen, d.h. die Tatsache, daß es sich um einen nach innen gerichteten Strom handelt, der durch Hyperpolarisierung aktiviert wird und den zuvor beschriebenen K⁺-Leitwert I_{K2} auf kuriose Weise ähnlich war. Es besteht ein wachsendes Interesse an diesem Strom, da er z.B. an der Erzeugung und Steuerung spontaner Aktivität des Herzens beteiligt ist.

Es wurden weitere Beweise für das Vorhandensein eines entsprechenden Stroms in zentralen Neuronen gefunden und erstmalig von Halliwell und Adams (1982) aufgeführt. Sie beobachteten in Pyramidenzellen des Hippocampus nach Hyperpolarisierung einen langsamen, nach innen gerichteten Strom, der als "queer"-Strom (I_{q}) bezeichnet wurde. In der Folgezeit wurden Ströme mit ähnlichen Eigenschaften in einer großen Vielzahl von neuronalen und nicht neuronalen Zellen gefunden und dieser hyperpolarisations-aktivierte Strom wurde schließlich als allgegenwärtiges Phänomen in Zellen des Nervensystems erkannt. Die Bezeichnung als "lₕ" hat sich für die Beschreibung dieses Stroms in der Zwischenzeit durchgesetzt.

Obwohl man zunächst annahm, daß die Aktivität der jeweiligen Iₕ-Kanäle nicht moduliert wird, zeigen immer mehr Daten, daß die Iₕ-Kanäle wichtige Ziele für Neurotransmitter und Botensysteme sind, was ihre wichtige physiologische Rolle bei der Steuerung zellulärer elektrischer Aktivitäten unterstreicht.

Mittlerweile weiß man, daß Iₕ signifikant zum Ruhepotential beiträgt, eine überschiessende Hyperpolarisierung begrenzt, die Form von Aktionsmustern (firing pattern) bestimmt und in der Erzeugung rhythmischer Oszillationen des Membranpotentials beteiligt ist. Iₕ-Ströme besitzen einige besondere Eigenschaften, die sie von anderen spannungsgesteuerten Ionenkanälen unterscheiden. Wie spannungsgesteuerte Na⁺, Ca²⁺ und bestimmte K⁺-Ströme, zeigen sie eine steile Spannungsabhängigkeit und aktivieren mit einem sigmoiden Zeitverlauf; sie werden jedoch durch Hyperpolarisation aktiviert und de-aktivieren mit einer sigmoiden Kinetik.

Die Aktivierung bei negativen Potentialen und die Blockierung durch Cs⁺-Ionen erinnert an einwärts gleichrichtende K⁺-Kanäle. Viele Eigenschaften von Iₕ unterscheiden sich jedoch deutlich von dieser K⁺-Kanalfamilie - Die Aktivierungskinetik ist langsamer, der Aktivierungsbereich ist positiver und unabhängig von der extrazellulären K⁺-Konzentration, der Leitwert ist im wesentlichen resistent gegen extrazelluläre Ba²⁺-lonen und die Iₕ-Kanäle sind nicht nur für K⁺-Ionen, sondern auch für Na⁺-Ionen permeabel. Anders als andere Kationenkanäle, wie z.B. ligandengesteuerte Kationenkanäle, sind die Iₕ-Kanäle sehr selektiv für Na⁺- und K⁺-Ionen und sie besitzen eine steile, spannungsabhängige Steuerung.

Besonders wichtig für die gegenwartige Forschung ist die Beteiligung der I-Kanale an der Schrittmacherfunktion im Herzmuskel. Die Schrittmacheraktivitat im Herzen ist auf spezialisierte Myozyten zurückzuführen, die in bestimmten Bereichen des Herzens (*sinus venosus*) lokalisiert sind, und die durch ihre Fähigkeit charakterisiert sind, spontan zu schlagen, selbst wenn sie vom Rest des Herzmuskels getrennt sind. In Schrittmacherzellen des Sinusknotens bei Säugern ergibt sich die spontane Aktivität aus einer typischen Phase ihres Aktionspotentials, der langsamen diastolischen Depolarisierung. Während dieser Phase, die der Diastole des Herzkontraktionszyklusses entspricht, depolarisiert die Membran nach der Beendigung des Aktionspotentials wieder langsam, bis der Schwellenwert für die Ausbildung eines neuen Aktionspotentials erreicht wird. So ist die diastolische Depolarisierung für die Initiierung des rhythmischen Verhaltens verantwortlich und charakterisiert Aktionspotentiale des Sinusknotens und anderer spontan aktiver Kardiozyten.

Neben der Erzeugung rhythmischer Aktivität ist die diastolische (oder Schrittmacher-) Depolarisierung an der Steuerung der Herzschlagfrequenz durch autonome Neurotransmitter beteiligt. Es ist bekannt, daß die Stimulierung des sympathischen und parasympathischen Nervensystems zur Beschleunigung bzw. Verlangsamung des Herzschlags führt.

In der Zwischenzeit ist bekannt, daß die Iₕ-Kanäle an dieser Schrittmacherfunktion beteiligt sind. Der Iₕ-Strom des Sinusknotens ist ein unspezifischer Kationenstrom, normalerweise getragen durch Na⁺ und K⁺, der langsam nach Hyperpolarisierung in einem Spannungsbereich aktiviert, der denjenigen der diastolischen Depolarisierung umfaßt. Die Iₕ-Merkmale sind gut geeignet, um einen Depolarisierungsprozeß zu erzeugen als Reaktion auf eine Hyperpolarisierung in einem Spannungsbereich bei dem der Iₕ-Kanal aktiviert wird.

Es ist bis jetzt jedoch noch nicht gelungen, Sequenzen von Genen zu identifizieren die für Iₕ-Ionenkanäle kodieren. Ferner stand bisher nicht genügend Kanalprotein zur Verfügung, um dieses biochemisch zu charakterisieren. Schließlich war bisher die pharmakologische Charakterisierung von Iₕ-Kanälen äußerst schwierig, weil die lₙ-Strome an ganzen Zellen die zusatzliche K⁺- und Na⁺-selektive Leitfahigkeiten besitzen, identifiziert und von den anderen Stromen experimentell isoliert wurden

Es war daher eine Aufgabe der vorliegenden Erfindung, die Nukleinsäure anzugeben, deren Verwendungsmöglichkeiten aufzuzeigen, sowie das Protein in einem funktionellen Zustand und in ausreichender Menge für biochemische Analysen und pharmazeutische Anwendungen bereitzustellen.

Diese Aufgabe wird durch den Gegenstand der unabhängigen Ansprüche gelöst. Vorteilhafte Weiterbildungen sind in den abhängigen Ansprüchen angegeben.

Die im folgenden verwendeten Begriffe haben folgende Bedeutung:

Unter "Iₕ-Ionenkanal" sollen hier solche Ionenkanäle verstanden werden, die (1) sich durch Hyperpolarisation öffnen und bei positiveren Spannungswerten (Vₘ ≥ -10 mV) geschlossen sind; (2) deren Aktivierung und De-Aktivierung mit einem relativ langsamen, sigmoidalen Zeitverlauf erfolgt; (3) nicht nur K⁺-Ionen, sondern auch Na⁺-Ionen leiten; (4) durch 0,1 - 3 mM extrazelluläres Cs⁺ fast vollständig blockiert werden und (5) durch zyklische Nukleotide, insbesondere cyclo AMP und cyclo GMP direkt moduliert werden.

Unter "stringenten Bedingungen" wird eine Hybridisierung in 0,1-5 x SSC, vorzugsweise 1-2 x SSC, bei 60-70°C, vorzugsweise 65°C, verstanden.

Unter "Bedingungen niedriger Stringenz" wird eine Hybridisierung mit 0,1-5 x SSC, vorzugsweise 1-2 x SSC bei 50-60°C, vorzugsweise bei 55°C, verstanden.

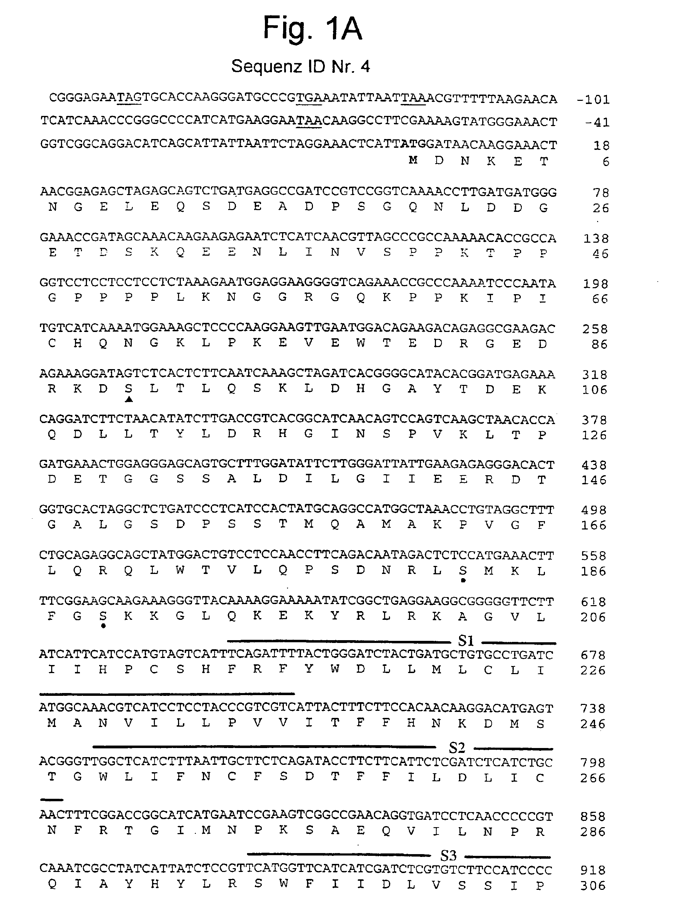

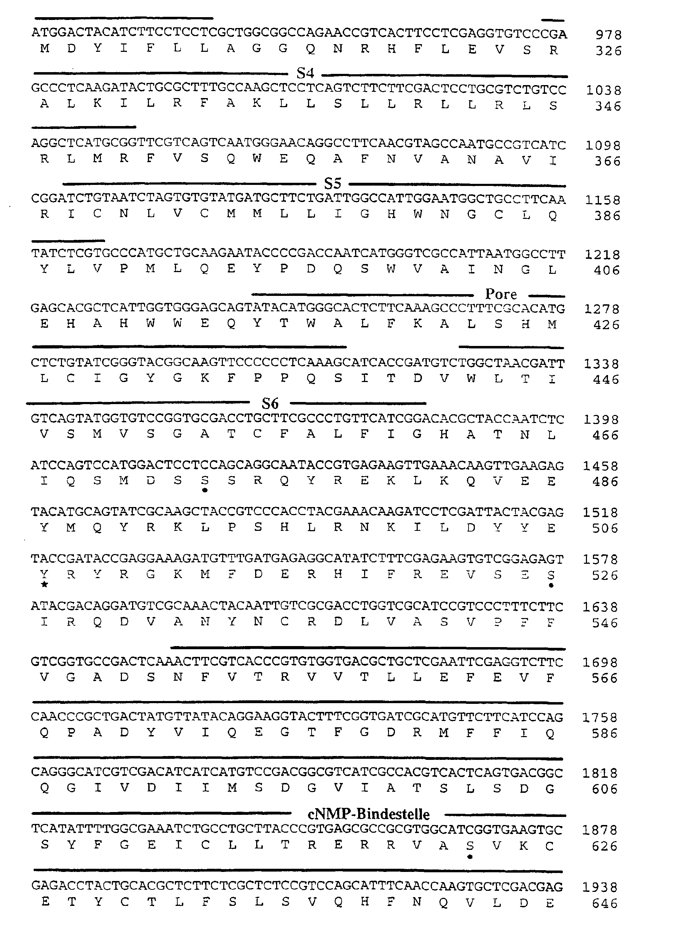

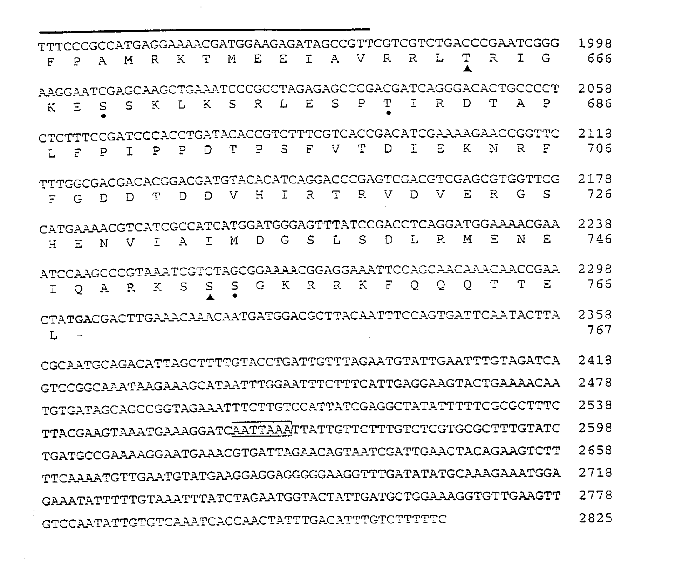

"Teile" des lₕ-Ionenkanals bedeutet einen Abschnitt der Proteinsequenz, der als antigene Determinante geeignet ist, beispielsweise ein Abschnitt von mindestens 6 Aminosäuren. Als Teil werden auch Abschnitte angesehen, die in Form von Domänen auftreten, wie beispielsweise die Abschnitte. S1, S2 etc.. wie in Figur 1 A angegeben Mit umfaßt sind Abschnitte des Ionenkanals, die sich von den in SEQ ID NO 1 bis 15 angegebenen DNA-Sequenzen die unter Verwendung des IUPAC-Kodes angegeben sind, durch Aminosäureaustausche, Deletionen und Additionen ableiten unter Beibehaltung der biologischen Funktion.

"Teil" davon im Zusammenhang mit der Nukleinsäure bedeutet ein mindestens 6 Nukleotide langes Fragment, vorzugsweise 12 Nukleotide, besonders bevorzugt 18 Nukleotide lang. Der Teil ist geeignet, über Oligonukleotidhybridisierung spezifisch (selektiv) mit der korrespondierenden Gesamtsequenz zu hybridisieren. Danach ist ein "Teil" der Nukleinsäure ein Abschnitt aus den Sequenzen gemäß SEQ ID NO 1 bis 15, der geeignet ist, selektiv mit einer der genannten Sequenzen zu hybridisieren.

"Selektiv" (spezifisch) bedeutet, daß eine Nukleinsäure unter den geeigneten Hybridisierungsbedingungen nur mit einer Nukleinsäure hybridisiert, wie durch eine der Sequenzen nach SEQ ID NO 1 bis 15 angegeben, während sie nicht mit einer anderen Nukleinsäure des jeweiligen Wirtsorganismus hybridisiert, mit dem sie üblicherweise assoziiert ist.

"Homologie", wie hierin verwendet, berechnet sich wie folgt: Es werden die Aminosäuren in den miteinander verglichenen Sequenzen oder Sequenzabschnitten gezählt, die entweder an der jeweiligen Position identisch oder ähnlich sind. Diese Zahl wird durch die Gesamtzahl der Aminosäurereste geteilt und mit 100 multipliziert. Daraus ergibt sich ein Prozentsatz der Sequenzähnlichkeit bzw. Homologie. Das unten aufgeführte Beispiel illustriert dies:
TWALFKALSHMLCIGYGKFPPQS
PDAFWWAWTMTTVGYGDMTPVG

Die Gesamtzahl der miteinander verglichenen Positionen beträgt 23 Reste; es gibt 7 identisch und 6 ähnlich besetzte Aminosäurepositionen. Deshalb ist die Homologie (7 + 6)/23 x 100 = 56,5 %. Ein Austausch von ähnlichen Aminosäuren wird auch als konservativer Austausch bezeichnet (vgl. Dayhoff et al., 1978).

Gemäß Anspruch 1 wird eine Nukleinsäure bereitgestellt, die die Sequenz SEQ ID NO 15 eines Iₕ-Ionenkanals umfaßt. Die hierzu komplementäre Nukleinsäure wird ebenfalls als erfindungsgemäße Ausführungsform betrachtet. Diese Nukleinsäure ist von einer humanen DNA abgeleitet.

Illustriert werden hier auch Sequenzen, die von einer Ratten-DNA abgeleitet sind, und durch die SEQ ID NO 2 sowie SEQ ID NO 8, SEQ ID NO 9, SEQ ID NO 13 und SEQ ID NO 14 beschrieben werden.

Desweiteren werden Sequenzen illustriert, die von einer Rinder-DNA abgeleitet sind und dies sind die Sequenzen gemäß SEQ ID NO 3 sowie SEQ ID NO 6, SEQ ID NO 7 und SEQ ID NO 12.

Weiterhin wird die Sequenz von einer Seeigel-DNA illustriert, die durch die Sequenz SEQ ID NO 4 beschrieben wird.

Weiterhin kann die illustrierte DNA von *Drosophila melanogaster* abgeleitet sein. Die vollständige Sequenz ist dann gemäß SEQ ID NO 5.

In einer besonders bevorzugten Ausführungsform sind Sequenzen enthalten in Ansprüchen 2-5 definiert sind.

Von der vorliegenden Erfindung mit erfaßt sind Abwandlungen der Sequenzen gemäß SEQ ID NO 15, die durch die Ansprüche definiert sind und sich beispielsweise durch die Degeneration des genetischen Kodes, Deletionen, Insertionen, Inversionen und weiterer Mutationen ergeben, wobei vorzugsweise die biologische Eigenschaft des kodierten Kanalproteins oder Teils davon beibehalten wird.

Die Erfindung betrifft ferner ein mRNA-Molekül, das eine Sequenz umfaßt, die mit einer der oben beschriebenen Sequenzen korrespondiert. Entsprechend umfaßt die Erfindung ein Polypeptid, das von der oben genannten Nukleinsäure kodiert wird.

Die oben beschriebenen Sequenzen können für ein Screening-Verfahren oder auch zur Verwendung in der Diagnose verwendet werden. In einem Screening-Verfahren ergibt sich durch die Identifizierung der Sequenz des Iₕ-Kanals die Möglichkeit, die Wirkung von Substanzen auf Ionenkanäle unter Verwendung dieser Sequenzen zu testen.

Ein solches Screening-Verfahren, kann beispielsweise die folgenden Schritte umfassen:
- Erzeugung homogener Kanalpräparate, beispielsweise durch Expression der oben genannten Nukleinsäure in einem geeigneten Wirt, wie beispielsweise Oocyten, Säugerzellen etc.,
- Testen von Substanzen an diesen Kanalpräparationen.

Dabei läßt sich durch Messen der Kanalaktivität unter der Einwirkung bzw. Abwesenheit von Testsubstanzen ermitteln, welche Substanzen zur Beeinflussung der Kanäle geeignet sind.

Die Erfindung betrifft auch einen Kit zur Durchführung eines solchen Screening-Verfahrens, das mindestens eine der oben beschriebenen Nukleinsäuren bzw. Polypeptide umfaßt.

Die Sequenzen können auch für die Diagnose verwendet werden, insbesondere für die Erkennung von Herz/Kreislaufstörungen.

Zur Diagnose wird vorzugsweise die Nukleinsäure des Patienten mit einem Sequenzabschnitt einer der oben beschriebenen DNAs und/oder RNAs in Berührung gebracht, so daß ein Signal erhalten wird, das die Anwesenheit und/oder Abwesenheit einer Ionenkanalnukleinsäuresequenz anzeigt. Durch die Wahl geeigneter Proben, z.B. kurzer Oligonukleotide, lassen sich auch Mutationen in den Ionenkanälen der Patienten nachweisen, was wiederum für die Differentialdiagnose hilfreich ist.

Die Erfindung betrifft ferner einen Kit zur Durchführung eines solchen Diagnoseverfahrens, das eine der oben beschriebenen Sequenzen umfaßt.

Es ist außerdem möglich, die oben beschriebenen Sequenzen für eine Behandlung und/oder Prophylaxe von Herz/Kreislaufstörungen sowie Bewußtseinsstörungen und Schmerzzuständen einzusetzen. In einer bevorzugten Ausführungsform können Medikamente bereitgestellt werden, um Herz/Kreislaufstörungen zu behandeln oder frühzeitig zu erhinner, die auf einer Fehlsteuerung des Sinusknotens beruhen. Weiterhin werden bevorzugt Bewußtseinsstörungen erkannt, die auf einer Fehlfunktion cortico-thalamischer Neuronen beruhen. So kann beispielsweise im Rahmen der Gentherapie ein voll funktionsfähiger Ionenkanal, wie von den hierin beschriebenen Nukleinsäuren kodiert, in einen Patienten eingebracht werden, um so einen nicht mehr funktionsfähigen Kanal zu ersetzen.

Schließlich betrifft die Erfindung eine pharmazeutische Zusammensetzung, die eine oder mehrere der oben beschriebenen Nukleinsäuren oder das oben beschriebene Polypeptid umfaßt. Eine solche pharmazeutische Zusammensetzung kann zur Behandlung von Herz/Kreislaufstörungen, insbesondere solchen, die auf einer Fehlsteuerung des Sinusknotens beruhen, sowie Bewußtseinsstörungen, insbesondere solchen, die auf einer Fehlfunktion in cortico-thalamischen Neuronen beruhen, eingesetzt werden.

Im folgenden soll die Erfindung anhand der Beispiele und der beiliegenden Figuren beschrieben werden.
Dabei zeigen die Figuren das Folgende:
**Figur 1A** zeigt die Nukleinsäure- und die abgeleitete Proteinsequenz des Kanals aus Seeigel *Strongylocentrotus purpuratus* (SPHI-Kanal).
**Figur 1B** zeigt das S4-Motiv dieses Kanalproteins, im Vergleich mit anderen bekannten Kanalsequenzen;
**Figur 1C** zeigt das Porenmotiv dieser Sequenz im Vergleich mit anderen Sequenzen anderer Kanäle;
**Figur 1D** zeigt die cNMP-Bindungsdomäne der cDNA des Iₕ-Ionenkanals im Vergleich mit anderen Sequenzen von Ionenkanälen;
**Figur 2A** zeigt den nach innen gerichteten Strom mit einer komplexen Wellenform der durch die hyperpolarisierenden Spannungsschritte von einer Haltespannung von +10 mV zu negativeren Testwerten ausgelöst wird;
**Figur 2B** zeigt die Gleichgewichtsstrom/Spannungs(I/V)-Beziehung, bestimmt am Ende eines hyperpolarisierenden Spannungspulses;
**Figur 2C** zeigt das Meßprotokoll für die Bestimmung der "instantaneous" I/V-Beziehung aus der Amplitude der Tail-Ströme;
**Figur 2D** zeigt die "instantaneous" I/V-Beziehung, die leicht auswärts gleichrichtend ist, mit einer Umkehrspannung Vᵣₑᵥ von -30 mV;
**Figur 2E** zeigt, daß der Zeitverlauf der "tail"-Ströme vom Zeitpunkt der Spannungsänderung abhängt;
**Figur 2F** zeigt die Spannungsabhängigkeit der relativen Wahrscheinlichkeit, daß der Kanal offen ist, Pₒ die aus der Amplitude von Tail-Strömen bei +10 mV bestimmt wurde, ähnlich wie die in Figur 2A dargestellten;
**Figur 3A** zeigt die Auslösung von großen Gesamtzell-Strömen durch Hyperpolarisierung in Anwesenheit von 1 mM cAMP, die sich mit Verzögerung entwickelten und langsam ein Gleichgewicht erreichten;
**Figur 3B** zeigt die Spannungsabhängigkeit von P₀, bestimmt aus normalisierten Ganzzell-"tail"-Strömen und "tail"-Strömen von inside-out-patches;
**Figur 3C** zeigt den schnellen Anstieg der Amplitude des nach innen gerichteten Stroms nach einer kurzen UV-Belichtung;
**Figur 3D** zeigt SPIH-Ströme von zellfreien Membranstücken ohne cAMP; und
**Figur 3E** das entsprechende wie Figur 3D jedoch mit cAMP;
**Figur 3F** zeigt die Abhängigkeit des Stroms von der cAMP-Konzentration, die durch eine einfache Bindungsisotherme mit K_{1/2} von 0,74 µM und einem Hillkoeffizienten, der sich nicht deutlich von eins unterscheidet, beschrieben werden kann;
**Figur 4A** und **4B** zeigen die Blockade der SPIH-Kanäle durch Cs* (Figur 4A Kontrolle, Figur 4B + 10 mM Cs⁺);
**Figur 4C** zeigt die IN-Beziehung in Gegenwart von 0 bis 10 mM Cs⁺.
**Figur 4D** zeigt einen Blot von normalisiertem Strom I/Iₘₐₓ (bei -70 mV) gegen Cs⁺.
**Figur 4E** zeigt die Ionenselektivität von SPIH-Kanälen auf Inside-Out-Patches, wobei 100 mM des Bad-K⁺ durch entsprechende Konzentrationen von Rb⁺, Na⁺, Li⁺ oder Cs⁺ ersetzt wurden;
**Figur 4F** zeigt die I/V-Beziehung unter den verschiedenen ionischen Bedingungen, die in Teil E gezeigt werden;
**Figur 4G** zeigt, daß die nach innen gerichteten Ströme fast vollständig unterbrochen waren, während sich die Amplituden der nach außen gerichteten Ströme nicht änderten, wenn das extrazelluläre Medium nur Na⁺ enthielt;
**Figur 4H** zeigt die I/V-Beziehung der Ströme aus Teil G bei verschiedenen K⁺-Konzentrationen;
**Figur 5A** zeigt einen Northern Blot der Kanalboten-RNA mit einem Haupttranskript von ungefähr 3,3 kb und einem kleineren Transkript von 2,9 kb;
**Figur 5B** zeigt eine lichtmikroskopische Aufnahme von Spermien aus *S*. *purpuratus* (rechtes Bild) und die entsprechende immunhistochemische Anfärbung mit einem Antikörper, der den SPIH-Kanal spezifisch erkennt (linkes Bild).
**Figur 5C** zeigt eine entsprechende Western-Blot-Analyse.
**Figur 6** zeigt eine schematische Darstellung des pc SPIH-Konstruktes, das für die heterologe Expression von SPIH in HEK 293-Zellen verwendet wurde. Der cDNA-Bereich ist als schraffierter Balken dargestellt, die angrenzenden Bereiche des Plasmidvektors (pcDNA I) als fette Linien. Aus der Lage des Promotors für die T7-Polymerase und der Schnittstellen im multiplen Klonierungsbereich kann die Orientierung der cDNA im Plasmidvektor abgeleitet werden. Die eingefügte Kozak-Sequenz ist mit K bezeichnet.

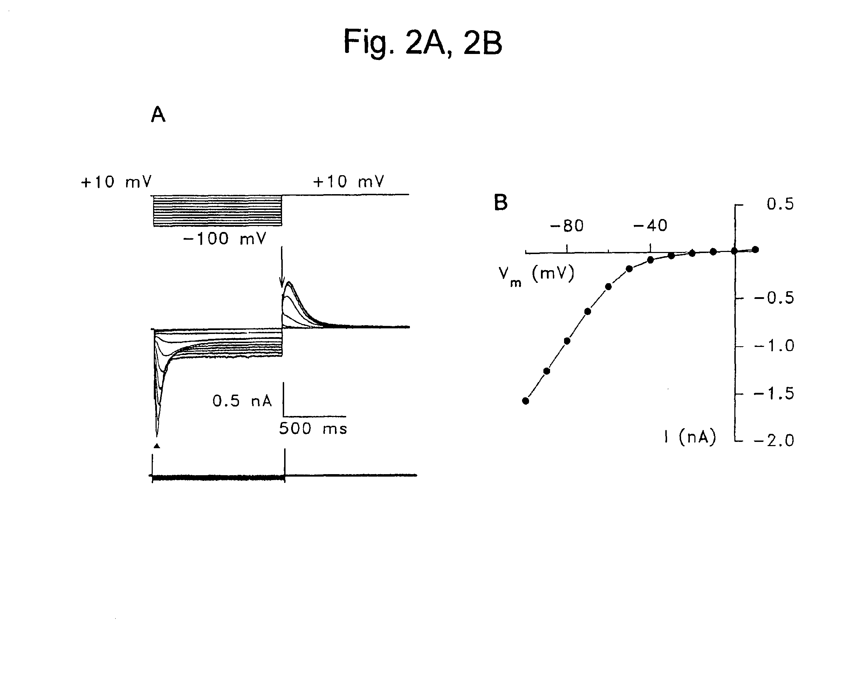

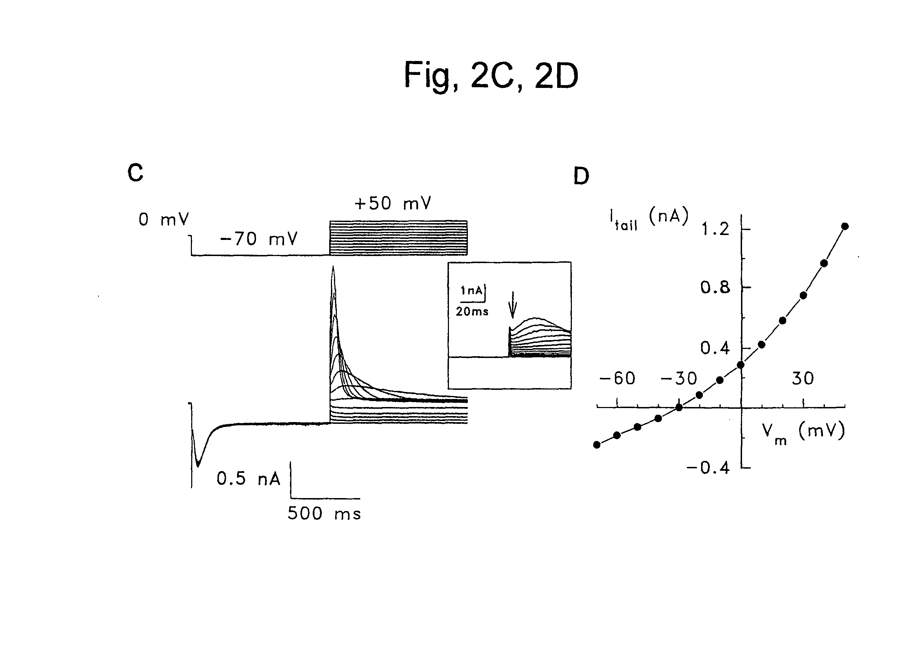

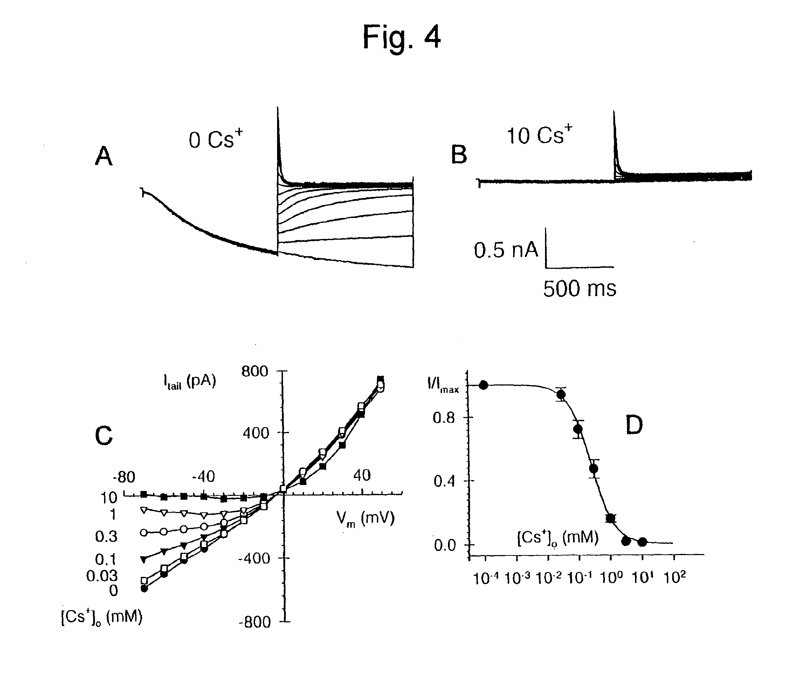

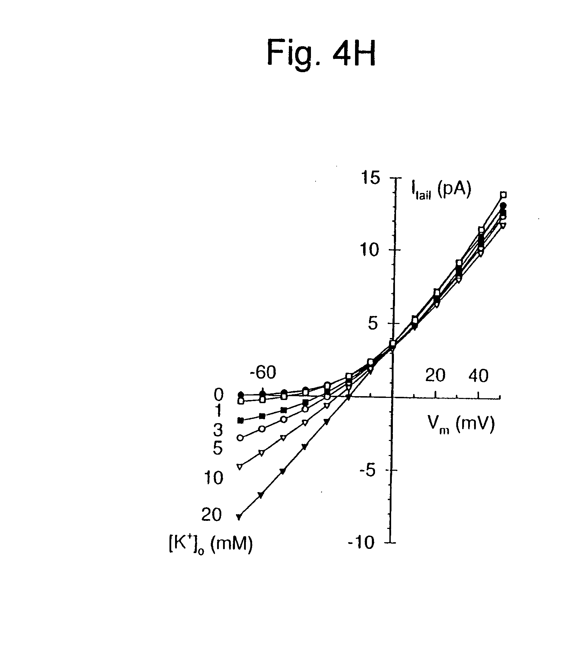

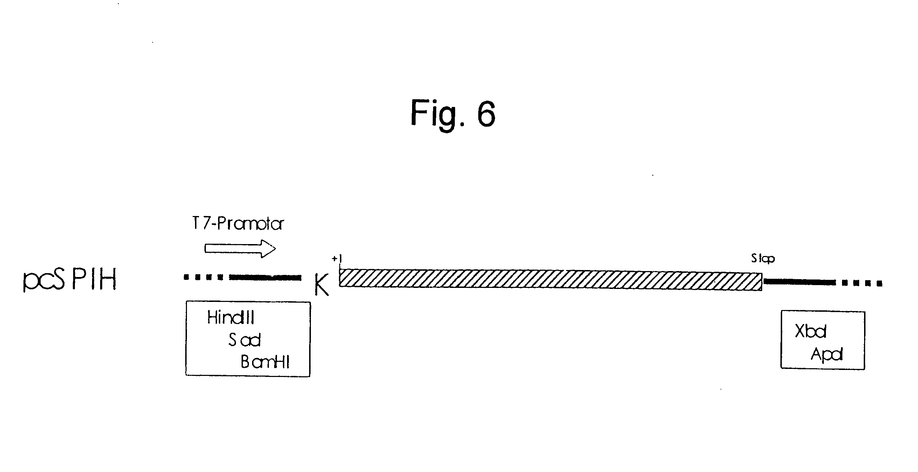

Ein repräsentativer Vertreter eines Ionenkanalproteins ist der Kanal aus Seeigel (SPIH). Die Kanalaktivität von HEK 293-Zellen, die mit dem pcSPHI-Konstrukt (Fig. 6) transfiziert worden waren, wurde mit Hilfe der Patch-Clamp-Methode in der Gesamtzellkonfiguration untersucht. Hyperpolarisierende Spannungsschritte zeigten einen einwärts gerichteten Strom mit einer komplexen Wellenform (vgl. Fig. 2A). Eine schnelle, nicht zeitaufgelöste Stromkomponente wurde gefolgt von einem zeitabhängigen Strom, der sich mit einer Verzögerung entwickelte und sich, nachdem das Maximum erreicht wurde, zu kleineren Amplituden abflachte, wenn die Testspannung Vₘ ≤ -30 mV betrug (Fig. 2A). Nachdem Vₘ auf +10 mV zurückgestellt wurde, entwickelten sich "tail"-Ströme, die auch einen komplexen Zeitverlauf zeigten. Die Steady-State-Beziehung zwischen Strom/Spannung (I/V), am Ende des hyperpolarisierenden Spannungspulses (Pfeil in Figur 2A), zeigte eine stark einwärts gerichtete Rektifizierung (Fig. 2B). Die "instantaneous" I/V-Beziehung wurde ermittelt aus der Amplitude der Tail-Ströme unter Verwendung eines anderen Protokolls für die Spannungsschritte (Fig. 2C). Die "instantaneous" I/V-Beziehung war leicht auswärts rektifizierend mit einer Umkehrspannung, Vᵣₑᵥ, von -30 mV (Fig. 2D). Die I/V-Beziehung wurde annähernd linear bei höheren [K⁺]ₒ, da der einwärts gerichtete Natriumstrom signifikant verstärkt wurde durch [K⁺]ₒ (siehe Figur 4H). Es kann gefolgert werden, daß die Ströme stark einwärts rektifizierend sind, da der SPIH-Kanal bei positiven Spannungen entweder geschlossen oder inaktiviert ist. Die Spannungsabhängigkeit der Offenwahrscheinlichkeit, Pₒ (Fig. 2F), wurde ermittelt aus der Amplitude der Tail-Ströme bei +10 mV (Fig. 2A). Die Spannung, V_{1/2}, bei der ein halbmaximaler Strom beobachtet wurde, lag bei -26,1 mV (7 Experimente). Somit ist der SPIH-Kanal inaktiv bei Spannungen ≥ +10 mV und wird durch Hyperpolarisation geöffnet. Diese Spannungsabhängigkeit erinnert an hyperpolarisations-aktivierte Ströme (Iₕ), die in verschiedenen Zellen vorkommen (DiFrancesco, 1990, 1993; Pape, 1996). Wegen seiner ungewöhnlichen Eigenschaften ist der Iₕ auch als "queer"- oder "funny"-Strom (I_{q} und I_{f}) bezeichnet worden. Der erfindungsgemäße Kanal wird (1) aktiviert bei hyperpolarisierenden Spannungen; (2) direkt moduliert durch zyklische Nukleotide; (3) blockiert durch millimolare Konzentrationen an extrazellulärem Cs⁺; (4) er ist kationselektiv mit einem P_{Na}/P_{K} von ~ 0,2 bis 0,4; und (5) die einwärts gerichteten Natriumströme sind sensitiv gegenüber [K⁺]ₒ. Die folgenden Untersuchungen zeigen, daß die genannten Merkmale auch bei dem heterolog exprimierten SPIH-Kanal auftreten.

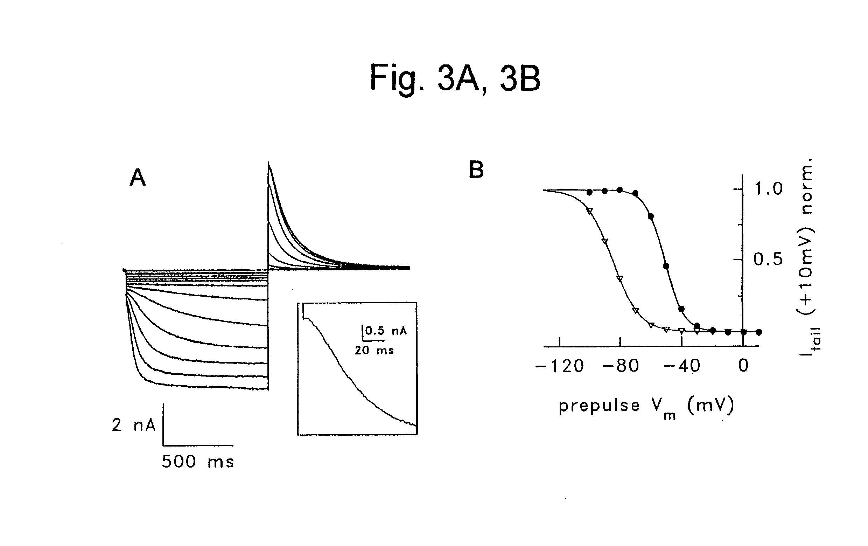

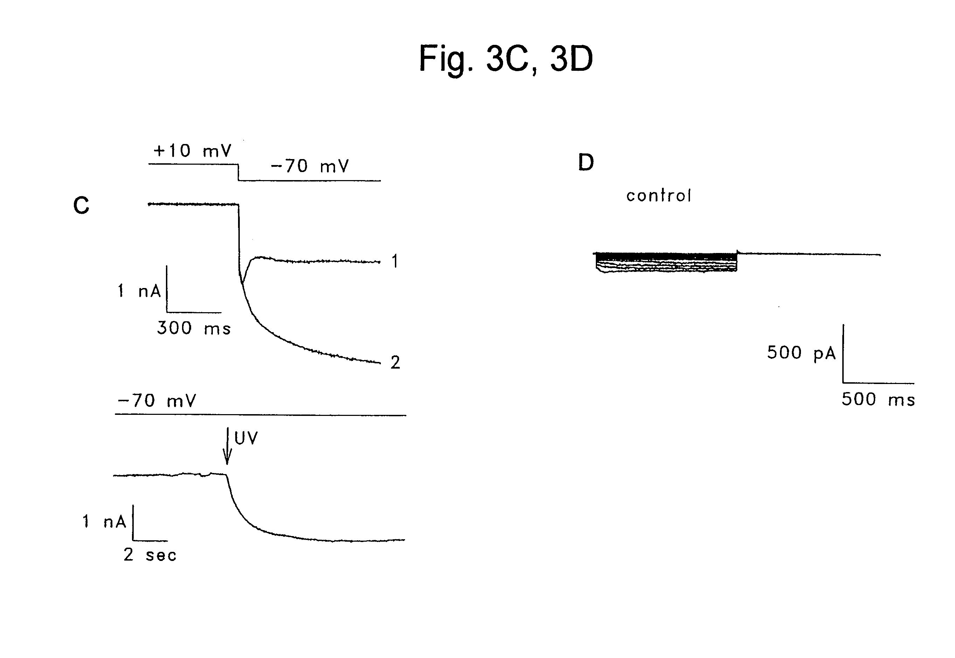

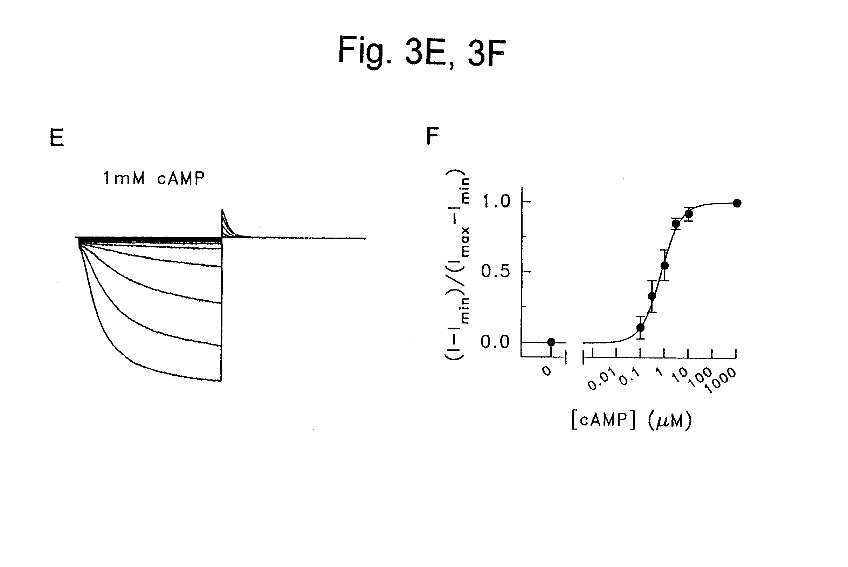

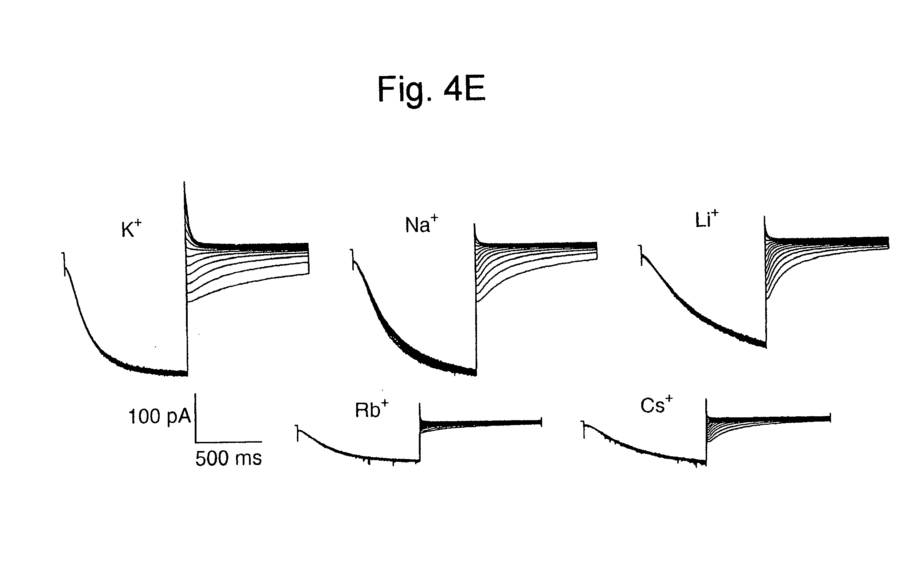

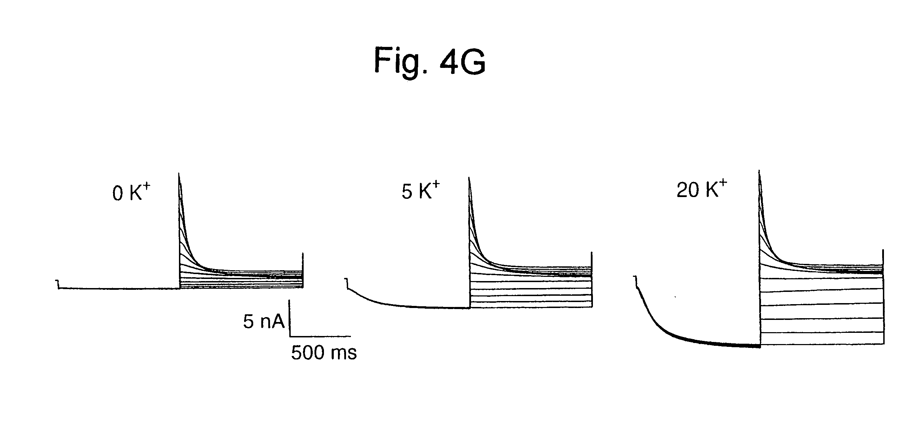

Wenn die Pipettenlösung 1 mM cAMP enthielt, führte die Hyperpolarisation zu starken Strömen, die sich mit einer Verzögerung entwickelten und langsam einen Steady-State-Zustand erreichten (Fig. 3A). Der sigmoidale Zeitverlauf des Stromes (siehe Fig. 3A, Kasten) ist charakteristisch für den zeitlichen Verlauf von Iₕ-Strömen aus Vertebraten. 1 mM cGMP in der Pipette änderte ebenfalls die SPIH-vermittelten Ströme. Die Spannungsabhängigkeit von Pₒ wurde mit Hilfe von Gesamtzell-Tail-Strömen ermittelt (Fig. 3B). Eine Anpassung an die Boltzmann-Gleichung ergab V_{1/2} = -50,8 mV. Die Dialyse der Zelle mit der Pipettenlösung beansprucht mehrere Minuten; somit könnten transiente Effekte von cAMP den Versuch beeinträchtigen. Es wurde deshalb eine Technik benutzt, die die rasche Fotofreisetzung von cAMP oder cGMP aus "caged" Derivaten einsetzt (vgl. Adams und Tsien, 1993; Hagen et al., 1996). Die Zellen wurden mit 100 µM "caged" cAMP dialysiert und die SPIH-Kanäle wurden durch Änderung der Vₘ von +10 mV auf -70 mV aktiviert; dann bewirkte ein kurzer Blitz von UV-Licht eine rasche Zunahme in der Amplitude des SPIH-vermittelten Einwärtsstroms (Fig. 3C). Die hyperpolarisationsaktivierten Ströme vor dem Blitz ähnelten Kontrollströmen (Fig. 3C, Spur 1), während die Amplitude und der Zeitverlauf der Ströme nach dem UV-Blitz (Fig. 3C, Spur 2) ähnlich waren zu denen, die in der Anwesenheit von cAMP aufgenommen wurden (Fig. 3E). Mit 100 µM "caged" cGMP in der Pipette änderten UV-Blitze von ähnlicher Dauer und ähnlicher Intensität nicht die SPIH-vermittelten Ströme. Ein Bindungsmotiv für zyklische Nukleotide legt nahe, daß cAMP direkt die Kanalaktivität verstärken könnte, ohne daß daran ein Phosphorylierungsmechanismus beteiligt ist. Zum Testen dieser Hypothese wurden die SPIH-Ströme an abgetrennten Membranflecken ohne (Fig. 3D) und mit cAMP (Fig. 3E) gemessen. cAMP (1 mM) steigerte die Amplituden der spannungsaktivierten Ströme bis auf das 20-fache. Der Stromanstieg durch cAMP war reversibel und erforderte kein Mg²⁺/ATP. Die Umspülung der abgetrennten Membranflecken mit Lösungen, die verschiedene cAMP-Konzentrationen enthielten, steigerte die SPIH-Ströme in einer dosisabhängigen Weise. Die Abhängigkeit des Stroms von der cAMP-Konzentration kann durch eine einfache Bindungsisotherme mit K_{1/2} von 0,74 µM und einem Hill-Koeffizienten, der sich nicht signifikant von eins unterscheidet, beschrieben werden (Fig. 3F). In den abgetrennten Membranflecken war V_{1/2} in Anwesenheit von cAMP ungefähr 35 mV stärker negativ ats V_{1/2}, das in der Gesamtzellkonfiguration gemessen wurde (Fig. 3B). Diese Beobachtung könnte darauf hindeuten, daß ein endogener Faktor, bereitgestellt durch die HEK293-Zelle, V_{1/2} mitbestimmt. cGMP-Konzentrationen bis zu 1 mM änderten die Amplitude der SPIH-Ströme nicht. Aus diesem Experiment kann geschlossen werden, daß cAMP, aber nicht cGMP, die SPIH-Kanalaktivität modulieren kann. Somit ist im Gegensatz zu CNG-Kanälen (Finn et al., 1996) SPIH unter der doppelten Kontrolle von Spannung und cAMP. Das Blockieren der SPIH-Kanäle durch extrazelluläres Cs⁺ wurde an "Outside-Out"-Membranen mit dem Spannungsprotokoll von Figur 2C untersucht. Cs⁺ blockierte den SPIH-Kanal in einer konzentrations- und spannungsabhängigen Weise. In der Anwesenheit von 10 mM Cs⁺ verschwanden die Einwärtsströme vollständig, während die auswärts gerichteten Tail-Ströme noch vorhanden waren (vgl. Fig. 4A und 4B). Die I/V-Relation in Anwesenheit von 0 bis 10 mM Cs⁺ ist in Figur 4C gezeigt. Der normalisierte Strom I/Iₘₐₓ (bei -70 mV) wurde gegen [Cs⁺] aufgetragen (Fig. 4D). Die Daten wurden mit einer Hemmkonstante Kᵢ von 245 µM und einem Hill-Koeffizienten von n = 1,2 angepaßt. Die Ionenselektivität des SPIH-Kanals wurde mit Inside-Out-Membranen bestimmt. Die Badlösungen enthielten immer 0,1 mM cAMP, um die Amplitude der Ströme zu erhöhen. Es wurden 100 mM K⁺ im Bad durch Rb⁺, Na⁺, Li⁺ oder Cs⁺ (Fig. 4E) ersetzt. Aus den jeweiligen Verschiebungen von Vᵣₑᵥ (Mittelwert von 3 bis 10 Experimenten) wurden die Permeabilitätsverhältnisse P_{K} : P_{Rb}: P_{Na}: P_{Li}: P_{Cs} als 1 : 0,7 : 0,26 : 0,15 : 0,06 berechnet. Die lonenselektivität von SPIH stimmt gut mit der lonenselektivität verschiedener Iₕ-Kanäle aus Wirbeltieren überein (Pape, 1996; Wollmuth und Hille, 1992). Wenn das extrazelluläre Medium nur Na⁺ enthielt, wurden die einwärts gerichteten Ströme nahezu vollständig eliminiert, während sich die Amplituden der auswärts gerichteten Ströme nicht wesentlich änderten (Fig. 4G). Die Erhöhung von [K⁺]ₒ auf 5 und 20 mM steigerte dramatisch die einwärts gerichteten Ströme. Diese Ergebnisse zeigen, daß der SPIH-Kanal wenig, wenn überhaupt, Natrium in Abwesenheit von Kaliumionen befördert.

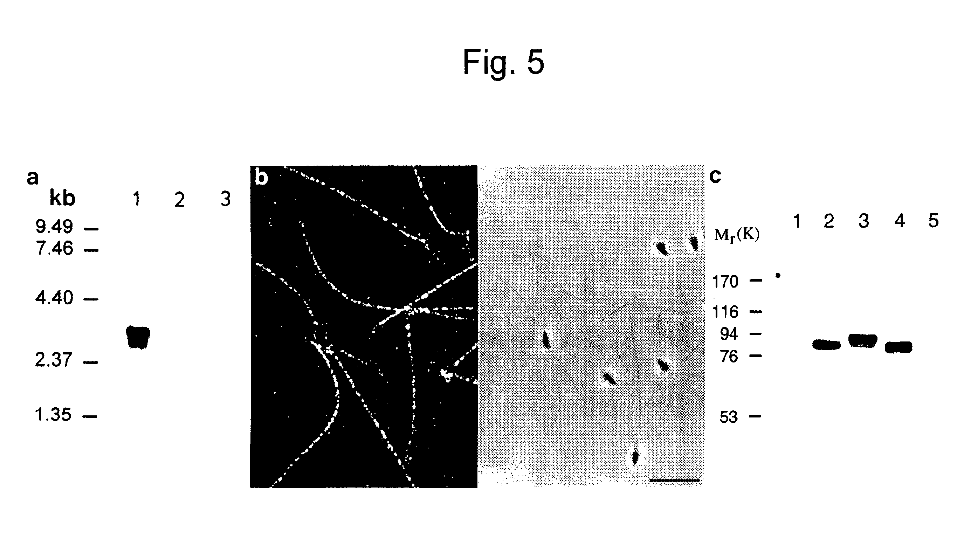

Die Expression von Messenger-RNA des Kanalproteins wurde mittels Northern Blots analysiert. Ein Haupttranskript von rund 3,3 kb und ein Nebentranskript von 2,9 kb wurden in Poly(A)⁺-RNA aus männlichen, aber nicht weiblichen Gonaden nachgewiesen (Fig. 5A). Die Größe der Transkripte stimmt gut mit der Größe der klonierten cDNA (3 kb) überein. Die SPIH-spezifische Probe hybridisierte nicht mit Poly(A)⁺-RNA, die aus dem Darmtrakt des Seeigels isoliert worden war (Fig. 5A). Die ausschließliche Expression von SPIH-mRNA in den männlichen Gonaden legt nahe, daß der Kanal in Spermien exprimiert wird. Diese Hypothese wurde getestet mit gereinigten Antikörpern FPc44K und FPc45K, die gegen ein Fusionsprotein der C-terminalen Domäne des Kanalpolypeptids (Reste 662-767) gerichtet waren. Die Antikörper wurden für die Western-Blot-Analysen (Fig. 5C) und die Immunzytochemie (Fig. 5B) verwendet. Beide Antikörper erkannten eine Hauptbande von Mᵣ ~ 92K in Western Blots von Flagellenmembranen, die aus Seeigelspermien gereinigt wurden (Fig. 5C, Spur 3). Membranen, die aus dem Spermienkopf gereinigt worden waren, wurden nicht von den Antikörpern erkannt (Fig. 5C, Spur 5). Dieses Ergebnis wurde durch Immunzytochemie mit einzelnen Spermien bestätigt. Der Antikörper FPc45K färbte nahezu ausschließlich das Spermienflagellum (Fig. 5B); die schwache Färbung einiger Kopfstrukturen repräsentiert vermutlich unspezifische Kreuzreaktivität des Antikörpers. Eine Bande von Mᵣ~ 88K wurde in Western-Blots von Membranen von transfizierten HEK293-Zellen beobachtet (Fig. 5C, Spur 2). Das Mᵣ des Kanalpolypeptids, exprimiert in HEK293-Zellen, ist nahezu identisch mit dem Mᵣ-Wert, wie er von der abgeleiteten Aminosäuresequenz zu erwarten ist (87,9K). In Membranen von nichttransfizierten HEK293-Zellen wurde kein 88K-Polypeptid durch den Antikörper nachgewiesen (Fig. 5C, Spur 1). Die Behandlung von Flagellenmembranen mit alkalischer Phosphatase erniedrigte den Mᵣ des nativen Polypeptids von ~ 92K auf 88K. Da native und heterolog exprimierte Polypeptide von ähnlicher Größe sind, trägt die klonierte cDNA die vollständige kodierende Sequenz von SPIH. Die geringe Abnahme des M, unter dephosphorylierenden Bedingungen zeigt an, daß das native Polypeptid in phosphorylierter Form mit einer leicht verringerten elektrophoretischen Mobilität vorliegt. In den meisten Dephosphorylierungsexperimenten war die Verschiebung von 92K auf 88K nicht vollständig, und es wurden zumindest zwei schwache Zwischenbanden beobachtet. Dieses Ergebnis legt nahe, daß das Kanalpolypeptid mehrfach phosphoryliert wird. Die SPIH-Sequenz trägt Sequenzmotive für die Phosphorylierung durch PKA, PKG, PKC und Tyrosinkinase (siehe Fig. 1A).

Die elektrophysiologischen Eigenschaften identifizieren SPIH unzweifelhaft als ein Mitglied der Iₕ-Kanalfamilie. Jedoch haben wir auch charakteristische Unterschiede zwischen SPIH und Iₕ-Kanälen aus Vertebraten beobachtet. Erstens: In Abwesenheit von cAMP ist der Strom von SPIH transient, während in Anwesenheit von cAMP der zeitliche Verlauf ähnlich ist wie bei Iₕ-Kanälen von Vertebraten. Zweitens: Die beträchtliche Zunahme des SPIH-Stroms durch cAMP resultiert primär aus einer Zunahme des Maximalstroms, während cAMP den kardialen Iₕ-Kanal so moduliert, daß V_{1/2} zu positiveren Werten hin verschoben wird (DiFrancesco, 1993), ohne die Maximalamplituden zu beeinflussen (siehe jedoch Ingram und Williams, 1996; Accili et al. 1997). Schließlich wird der kardiale Iₕ auch durch mikromolare cGMP-Konzentrationen moduliert (DiFrancesco und Tortora, 1991), während SPIH diesen Effekt nicht zeigt. Der SPIH-Kanal zeigt große Ähnlichkeit sowohl mit den spannungsgesteuerten K⁺-Kanälen als auch den CNG-Kationenkanälen. Deshalb bilden die Iₕ-Kanäle eine eigene Klasse innerhalb der Superfamilie der spannungsgesteuerten Kanäle. SPIH besitzt ein charakteristisches Motiv eines Spannungssensors (S4), wie die K⁺-, Na⁺- und Ca²⁺-Kanäle, die durch Depolarisation geöffnet werden. Obwohl es a priori keinen Grund dafür gibt, das S4-Motiv als Spannungssensor in einem durch Hyperpolarisation aktivierten Kanal auszuschließen, ist doch der Mechanismus der Aktivierung wie in HERG-K⁺-Kanälen (Trudeau et al., 1995; Smith et al., 1996) wahrscheinlicher. Von der stark einwärts gerichteten Rektifizierung von HERG ist gezeigt worden, daß sie das Ergebnis der Inaktivierung ist, die die Kanäle bei positiven Spannungen schließt, aber die Kanäle erholen sich rasch von der Inaktivierung bei negativen Spannungen. In HERG-Kanälen ist die Inaktivierung viel schneller als die Aktivierung und deshalb kinetisch nicht sichtbar (Smith et al., 1996). Gemeinsam mit CNG-Kanälen besitzt SPIH eine zyklische-Nukleotide-bindende Region, und seine Eigenschaften werden durch cAMP moduliert. Wahrscheinlich verstärkt cAMP die SPIH-Aktivität durch Binden an die stark konservierte zyklische-Nukleotide-bindende Region. In CNG-Kanälen ist für die hohe Selektivität für cGMP gezeigt worden, daß diese mit einem Thr-Rest (T363 in der α-Untereinheit des Stäbchen-Photorezeptors: Altenhofen et al., 1991) und einem Asp-Rest (D604 in rCNGα; Varnum et al., 1995) einhergeht. An den entsprechenden Positionen besitzt der SPIH-Kanal Val- und Ile-Reste; vermutlich kontrollieren diese Positionen auch die Ligandenselektivität in SPIH. Die physiologische Bedeutung der Iₕ-Kanäle in Flagellenmembranen von Spermien könnte wie folgt erklärt werden: Die Stimulation von *S*. *purpuratus*-Spermien mit dem chemotaktischen Pepitd "Speract" ruft eine Hyperpolarisation hervor (Lee und Garbers, 1986; Garbers, 1989), von der angenommen wird, daß sie auf der Öffnung eines K⁺-Kanals beruht (Babcock et al., 1992). Bei höheren Peptidkonzentrationen folgt der Hyperpolarisation eine Depolarisation (Babcock et al., 1992). Zwei (oder mehr) lonenkanal-Typen mit verschiedener Selektivität und Pharmakologie könnten zu der "Speract"-induzierten Depolarisation beitragen (siehe Darszon et al., 1996). Einer dieser Kanäle besitzt eine schwache K⁺-Selektivität (P_{Na}/P_{K} ≅ 0,2) und eine extrem niedrige Pₒ (bei Vₘ = 0 mV), die stark erhöht wird durch cAMP, aber nicht durch cGMP (Labarca et al., 1996). Diese Beobachtungen legen nahe, daß dieser Kanal in der Tat SPIH ist. Die "Speract"-induzierte Hyperpolarisation könnte die SPIH-Kanalaktivität initiieren, die noch weiter gesteigert werden könnte durch eine gleichzeitige Erhöhung der cAMP-Spiegels (Hansbrough et al., 1980), mit Hilfe einer spannungsabhängigen Adenylatcyclase (Beltrán et al., 1996). Bei der gegebenen ionischen Zusammensetzung von Meerwasser und einem P_{Na}/P_{K} von 0,2 bis 0,4 könnte das Öffnen des SPIH-Kanals und der anschließende Na⁺-Influx die "Speract"-induzierte Depolarisation bewirken. Es kann auch vernünftigerweise angenommen werden, daß die Iₕ-Kanäle, wie in Herzzellen oder thalamische Neuronen, an der Erzeugung von Oszillationen der Membranspannung beteiligt sind und dadurch die Oszillation von Ca²⁺ im Flagellum hervorrufen (Suarez et al., 1993). Die Änderung der [Ca²⁺]ᵢ könnte das Schlagmuster des Flagellums ändern und so zu der chemotaktischen Antwort beitragen.

Die nachfolgenden Beispiele illustration die Erfindung.

### BEISPIELE

### Methoden

### Isolierung der cDNA-Klone

Mit zwei degenerierten Primern (# 1764 und # 1772) wurde eine PCR auf Einzelstrang-cDNA (aus Seeigelgonaden, *Drosophila melanogaster,* Rinderretina, olfaktorischem Gewebe der Ratte) oder auf cDNA-Bibliotheken (aus humanem Thalamus bzw. Herz) durchgeführt. Ein 100 µl PCR-Ansatz hatte folgende Zusammensetzung: 3-10 ng Erststrang-cDNA bzw. ca. 10⁵ pfu der cDNA-Bibliotheken, jeweils 1,6 µg der degenerierten Primer, 1 x PCR-Puffer, 2 mM dNTP, 1 U PrimeZyme (Biometra). Der PCR-Ansatz wurde zunächst 2 min bei 94°C denaturiert und dann 45 Zyklen folgendermaßen inkubiert:

| | |
|---|---|
| Denaturierung: | 94°C, 45 sec |
| Hybridisierung: | 48°C, 45 sec |
| Polymerisierung: | 72°C, 40 sec |

Die Sequenzen der degenerierten Primer sind (in 5'→3'-Richtung):
# 1764: CTGACTGCAGARGTNTTYCARCCNGGNGA (SEQ ID NO 16)
# 1772: ATCGGAATTCNCCRAARTANGANCCRTC (SEQ ID NO 17)

Die mit den Primern # 1764 und 1772 amplifizierten PCR-Fragmente wurden radioaktiv markiert und als Sonden benutzt, um cDNA-Bibliotheken unter hoher Stringenz nach den vollständigen cDNAs zu durchsuchen. Der Teilklon HHIH (SEQ ID NO 11) wurde durch niedrig-stringente Hybridisierung isoliert. Die Hybridisierungsbedingungen waren:

| | hohe Stringenz | niedrige Stringenz |
|---|---|---|
| Prähybridisierung | 5 x SSC⁽¹⁾, 5 x Denhardt's⁽²⁾, | 5 x SSC⁽¹⁾, 5 x Denhardt's⁽²⁾, |
| | 0,1 % SDS, 0,1 mg/ml Heringssperma-DNA, 1-2 h, 65°C | 0,1 % SDS, 0,1 mg/ml Heringssperma-DNA, 1-2 h, 55°C |
| Hybridisierung | Prähybridisierungslösung mit 50-100 ng 32P-markierter DNA | Prähybridisierungslösung mit 50-100 ng 32P-markierter DNA |
| | (1x10⁶ cpm/ml), 12-14 h, 65°C | (1x10⁶ cpm/ml), 12-14 h, 65°C |
| Waschen | 1 x SSC(1), 0,1 % SDS | 2 x SSC(1), 0,1 % SDS |
| | 2 x 30 min, 65°C | 2 x 30 min, 55°C |

| | | |
|---|---|---|
| ⁽¹⁾ 1 x SSC 150 mM NaCl, 15 mM Na-Citrat, pH 7,0 ⁽²⁾ 1 x Denhardt's Ficoll, Polyvinylpyrrolidon, Rinderserum-Albumin (je 0,2 g/l) | | |

Die positiven Phagen wurden vereinzelt und die cDNA durch "in vivo excision" (bei λZAPII-Phagen) in pBluescriptSK-Derivate überführt. Aus λgt11-Phagen wurde die cDNA mit EcoR1 ausgeschnitten und in pBluescriptSK-Plasmid-DNA subkloniert. Die DNA wurde mit der Didesoxy-Kettenabbruchmethode (Sanger et al., 1977) sequenziert.

### Northern und Western Blot

Poly(A)⁺RNA, isoliert aus verschiedenen Geweben des Seeigels, wurde durch Northern Blot analysiert. Jede Spur enthielt ungefähr 10 µg Poly(A)⁺RNA. Der Blot wurde mit einem ³²P-markierten 1074 bp cDNA-Fragment (Nukleotidpositionen) bei 42°C, 5 x SSC und 50 % Formamid hybridisiert. Ein C-terminaler Bereich des SPIH-Polypeptids wurde als Fusionskonstrukt mit dem Maltosebindungsprotein exprimiert. Das gereinigte Fusionsprotein wurde verwendet, um die polyklonalen Antikörper FPc44K und FPc45K zu erzeugen die Antikörper wurden aus Kaninchenserum durch Affinitätschromatographie unter Verwendung des Fusionsproteins gereinigt Spermienflagellen wurden vom Kopf gemäß Darszon et al. (1994) abgetrennt. Gereinigte Flagellen und Kopfmembranen wurden in einem Lösungspuffer homogenisiert, der 150 mM NaCl, 1 mM MgCl₂, 20 mM Hepes bei pH 7,5, 0,1 mM EGTA und 0,5 % Triton X100 enthielt, gefolgt von einer Zentrifugation für 60 Minuten bei 40000 Upm. Dieses Verfahren wurde 2 x wiederholt. Transfizierte HEK293-Zellen wurden in einem Lysepuffer homogenisiert (10 mM Hepes, 1 mM DTT und 1 mM EDTA bei pH 7,4), 5 x gefriergetrocknet (in flüssigem N₂) und schließlich 10 min bei 55000 Upm zentrifugiert. Das Membranpellet wurde in dem Lösungspuffer gelöst. Flagellenmembranproteine wurden mit einer Einheit alkalischer Phosphatase in Lösungspuffer 30 bis 60 min bei 30°C dephosphoryliert. Die Membranproteine wurden durch SDS-PAGE getrennt, auf Immobilon-Membranen übertragen und mit den polyklonalen Antikörpern markiert. Die Immunoreaktivität wurde durch das ECL-Detektionskit (Amersham) sichtbar gemacht. Die Immunozytochemie an einem einzelnen Spermium wurde wie vorher beschrieben durchgeführt (Weiner 1997).

### Elektrophysiologie

cDNA, die das SPIH-Polypeptid kodierte, wurde transient in HEK293-Zellen exprimiert, wie früher beschrieben (Baumann et al., 1994). SPIH-gesteuerte Ströme wurden mit der Patch-clamp-Methode in der Gesamtzellkonfiguration sowie zellfreien Membranflecken aufgenommen. Die Zusammensetzung verschiedener Bad- und Pipettenlösungen sind in den Legenden der Figuren (s.u.) angegeben. Die Kanäle wurden durch Abstufung der Membranspannung von +10 mV auf verschiedene negative Spannungswerte aktiviert. Leckströme wurden unter Verwendung eines P/8-Protokolls abgezogen. Die Spannungsabhängigkeit der Wahrscheinlichkeit, daß der Kanal offen ist, wurde aus Tail-Strömen bei + 10 mV bestimmt. Die Blockade des SPIH-Kanals durch Cs⁺ wurde mit Outside-Out-Membranflecken in Gegenwart von 1 mM cAMP in einer Pipettenlösung untersucht. Die Lösungen in dem Bad enthielten 0,03 bis 10 mM CsCl. Relative lonenpermeabilitäten wurden aus der respektiven Verlagerung von Vᵣₑᵥ berechnet, das an zellfreien Inside-Out-Membranflecken gemessen wurde, wenn 100 mM K⁺ im Bad durch Na⁺, Li⁺, Rb⁺ oder Cs⁺ ersetzt worden waren. Experimente mit "caged" cAMP oder "caged" cGMP wurden wie vorher beschrieben durchgeführt (Hagen et al. 1996).

Im folgenden werden die Ergebnisse dieser Experimente genauer beschrieben.

In **Figur 1A** zeigt die Nukleinsäure-Sequenz und die abgeleitete Aminosäuresequenz des Iₕ-Kanals aus Seeigel (SPIH). Nukleotide sind in 5'→3'-Richtung numeriert, wobei +1 dem ersten Nukleotid des Startkodons (ATG) des offenen Leserahmen entspricht. Nukleotide 5'-wärts von Nukleotid +1 sind mit negativen Zahlen bezeichnet. Die abgeleitete Aminosäuresequenz (Ein-Buchstaben-Kode) ist unter der Nukleinsäure-sequenz angegben und ebenfalls nummeriert. Das Startkodon (ATG), das entsprechende Methionin und das Stopkodon (TGA; Pos. 2302-2304) sind fett gedruckt. Stopkodons im gleichen Leserahmen vor dem Startkodon sind unterstrichen. Das Polyadenylierungssignal an Position 2501-2507 ist umrahmt. Die Lage der transmembranalen Segmente S1-S6, der Poren-bildenen Region und der Bindestelle für zyklische Nukleotide (cNMP-Bindestelle) ist durch Balken über der Nukleinsäure-sequenz gekennzeichnet. Die Grenzen dieser Bereiche wurden durch Sequenzvergleich mit anderen spannungsabhängigen K⁺-Kanälen, EAG-K⁺-Kanälen und CNG-Kanälen festgelegt. Konsensussequenzen für Phosphorylierung durch cAMP/cGMPabhängige Kinasen sind durch Dreiecke (Δ) markiert. Konsensussequenzen für Phosphorylierung durch Proteinkinase C sind durch Kreise (•) und durch Tyrosinkinase durch einen Stern (*) markiert. Die SPIH-Sequenz (SEQ ID NO 4) kodiert für ein Protein von 767 Aminosäuren mit einem errechneten Molekulargewicht von 87.937 Da.

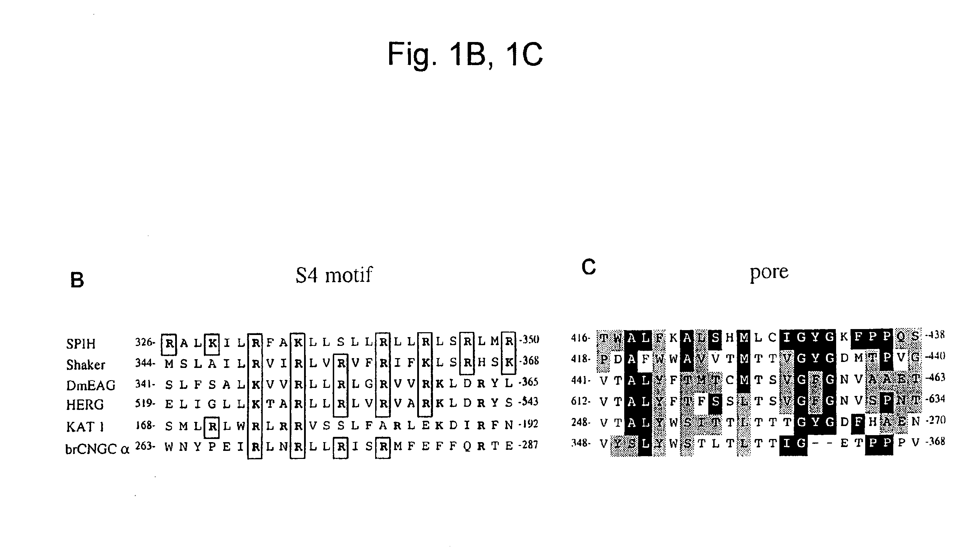

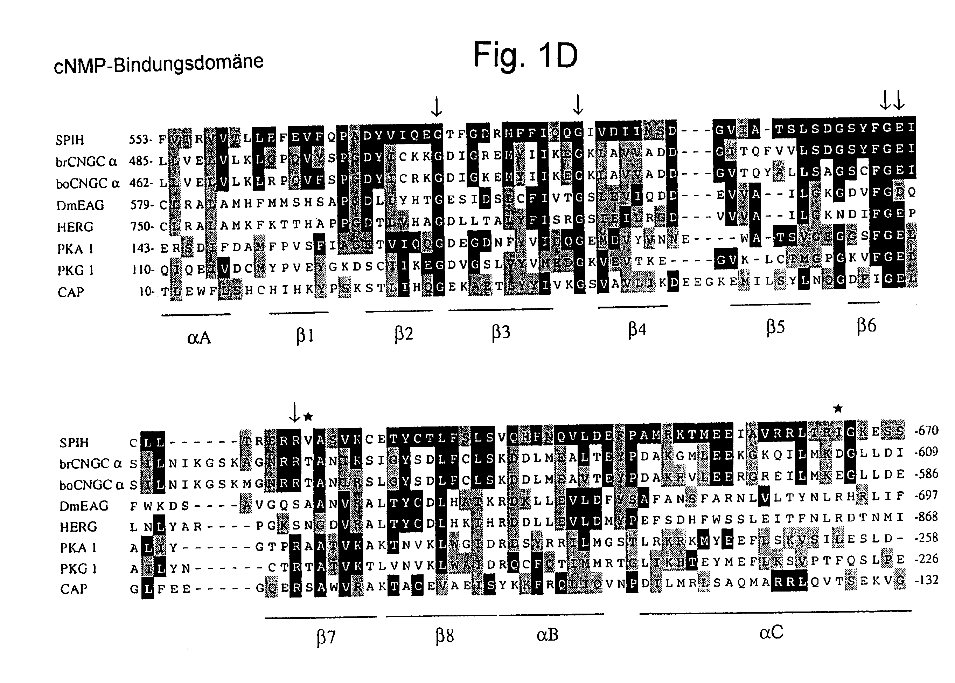

**Figur 1B** zeigt einen Vergleich der Spannungssensor(S4)-Motive des Iₕ-Kanals aus Seeigel und anderer Kanäle. Arg- oder Lys-Reste in regelmäßigen Abständen sind durch Einrahmung hervorgehoben. Andere positiv geladene Reste sind in Fettdruck.

Shaker (Pongs et al., 1988), K⁺-Kanal, kodiert durch das *Drosophila* Shakergen:
DmEAG (Warmke et al., 1991), *Drosophila* EAG-Kanal;
HERG, menschliches EAG-verwandtes Gen (Warmke und Ganetzky. 1994):
KAT1 (Anderson et al., 1992). K⁺-Kanal von *Arabidopsis thaliana*;
brCNGCα (Kaupp et al., 1989), α-Untereinheit des cyclischen nukleotidgesteuerten Kanals von bovinen Stäbchenphotorezeptoren.

In der **Figur 1C** wird das Porenmotiv von SPIH mit den Porenmotiven anderer Mitglieder der Superfamilie der spannungs- und cyclischen nukleotidgesteuerten Ionenkanäle dargestellt:

Die Reste, die mit den korrespondierenden Aminosäuren in SPIH identisch oder ähnlich sind, werden durch einen schwarzen oder grauen Hintergrund hervorgehoben.

Die **Figur 1D** zeigt einen Sequenzvergleich von cNMP-Bindungsdomänen. boCNGCα, die α-Untereinheit des CNG-Kanals von bovinen olfaktorischen Neuronen (Ludwig et al., 1990); PKA1, die cAMP-Bindungsstelle 1 der Proteinkinase A (Titani et al., 1984); die cGMP-Bindungsstelle 1 der Proteinkinase G (Takio et al., 1984); CAP, das katabolite Aktivatorprotein (Aiba et al., 1982). Reste, die in zyklischen Nukleotidbindungsmotiven hochkonserviert sind, sind durch Pfeile angezeigt; Reste, die die Ligandenselektivität in brCNGCα bestimmen, sind durch einen Stern angegeben. Vorhersagen über die Ausbildung von Sekundärstrukturen, die aus der cAMP-Bindungsdomäne von CAP abgeleitet wurden, werden als Balken unterhalb der Sequenz angegeben.

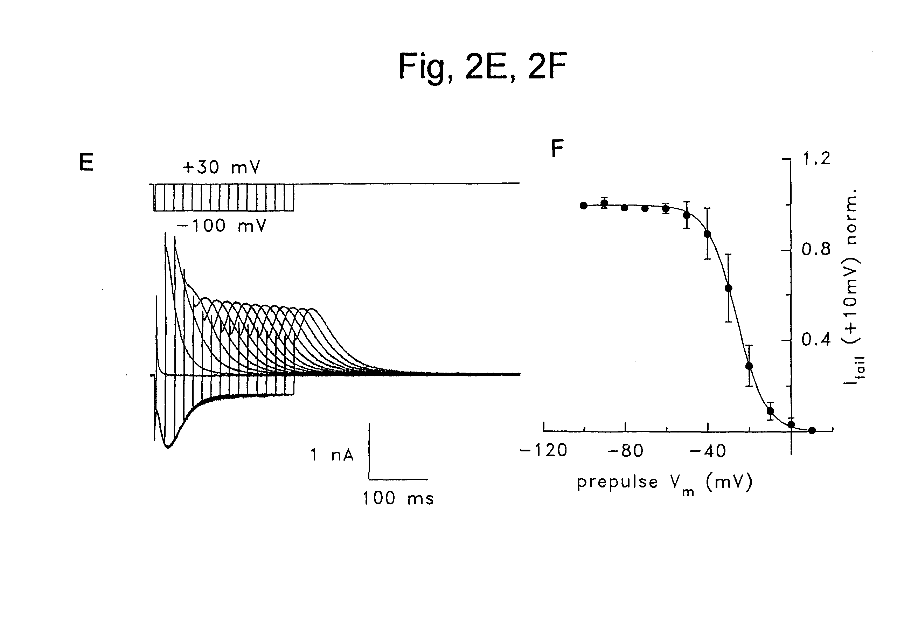

**Figur 2** zeigt die elektrophysiologische Charakterisierung des SPIH-Kanals.

In **Figur 2A** ist der Strom dargestellt, der von transfizierten HEK293-Zellen in der Gesamtzellkonfiguration aufgezeichnet wurde. Der Strom wurde durch Abstufung der Spannung von einem Haltewert bei ±10 mV auf verschiedene Testwerte von -100 mV bis +10 mV in Abständen von 10 mV aktiviert. Tail-Ströme wurden durch Abstufung der Spannung vom Testwert zurück zu +10 mV aufgezeichnet. Die HEK293-Zellen wurden mit einer Badlösung umspült, die folgendes enthielt (mM): 135 NaCl, 5 KCI, 1,8 CaCl₂, 2.8 MgCl₂ und 5 Hepes-NaOH bei pH 7,4; die Pipettenlösung enthielt die folgenden Substanzen (mM): 126 KCl, 10 Hepes-KOH, 10 EGTA bei pH 7.4.

In **Figur 2B** ist Spannung-Strom(I/V)-Beziehung aufgetragen, gemessen unter Gleichgewichtsbedingungen zur Zeit des in Figur 2A durch den Pfeilkopf angezeigten Zeitpunkts.

**Figur 2C** zeigt das Meß-Protokoll, mit dem die "instantaneous" I/V-Beziehung bestimmt wurde; die Spannung wurde zunächst von einem Haltewert von 0 mV auf - 70 mV abgestuft, gefolgt von Stufen auf Testwerte im Bereich von +50 mV bis -70 mV in 10 mV-Abständen.

**Figur 2D** zeigt dann den Plot der "instantaneous" I/V-Beziehung, gemessen zur Zeit wie in Figur 2C (eingerahmtes Bild) durch den Pfeil angezeigt.

**Figur 2E** zeigt, daß der Zeitverlauf der "tail"-Ströme von dem Zeitpunkt abhängt, zu dem die Spannung auf +30 mV zurückgesetzt wird.

**Figur 2F** zeigt die Spannungsabhängigkeit der relativen Offenwahrscheinlichkeit, Pₒ, des Kanals. Die Tail-Stromamplituden (Pfeil in Teil a) wurden auf den maximalen Strom normalisiert. Die Mittelpunktspannung, V_{1/2} betrug -26,1 mV. Die effektive Ladungsmenge, Q, die während des Kanalschaltens fließt, beträgt 3.5 Elementarladungen. Sie wurde aus einer Anpassung der Boltzmann-Funktion an die Daten erzielt. Mittel von 7 Experimenten.

In **Figur 3** ist die Modulation von SPIH-Kanälen durch zyklische Nukleotide angegeben.

**Figur 3A** zeigt den Gesamtzell-SPIH-Strom in Gegenwart von 1 mM cAMP. Das Spannungsstufenprotokoll ist dasselbe wie in Figur 2A. Das Bad enthielt (mM): 135 NaCl. 5 KCl. 1.8 CaCl₂, 2.8 MgCl₂ und 5 Hepes-NaOH bei pH 7,4: die Pipettenlösung enthielt (mM) 126 KCI, 10 Hepes-KOH, 10 EGTA bei pH 7,4 und 1 mM cAMP. Das eingerahmte Bild zeit eine Vergrößerung, an der der sigmoidale Zeitverlauf besonders gut zu erkennen ist.

**Figur 3B** zeigt, die Spannungsabhängigkeit der relativen Pₒ, abgeleitet von normalisierten Gesamtzell-Tail-Strömen bei +10 mV (●) und von Tail-Strömen, die von Inside-Out-Patches aufgezeichnet wurden (Δ). Eine durchgezogene Linie stellt einen Fit der Boltzmanngleichung an die Daten dar. V_{1/2} für die Gesamtzellströme von Teil A betrug -50,8 mV und für die Inside-out-patch-Ströme von Teil E betrug sie -84,7 mV; die Q-Werte betrugen 3.8 bzw. 2.7.

**Figur 3C** zeigt die Modulierung von Gesamtzell-SPIH-Strömen durch die Photolyse von "caged" cAMP Die Pipettenlösung enthielt 100 µM "caged" cAMP. Der SPIH-Strom wurde durch Spannungssprünge von +10 mV auf -70 mV aktiviert bevor der UV-Blitz gegeben wurde (Spur 1) und nach drei aufeinanderfolgenden UV-Blitzen (Spur 2). Der Zeitverlauf des Blitz-vermittelten Anstiegs des Stromes bei -70 mV ist darunter gezeigt.

Die **Figuren 3D und E** zeigen spannungsaktivierte SPIH-Ströme in Inside-Out-Membran-Patches ohne cAMP (D) und in Gegenwart von 1 mM cAMP (E) im Bad. Das Spannungsstufenprotokoll wurde wie in Figur 2A durchgeführt. Die Pipetten und Badlösungen enthielten (mM): 126 KCl, 10 Hepes-KOH, 10 EGTA bei pH 7,4 und 1 mM cAMP (Bad).

Aus **Figur 3F** ist die Abhängigkeit der SPIH-Stromamplitude von der cAMP-Konzentration ablesbar; die cAMP-Konzentrationen waren wie folgt (µM): 0.1; 0.3; 1; 3: 10 und 1000. Eine durchgezogene Linie zeigt eine Anpassung der Hill-Gleichung an die Daten; K_{1/2} = 0,74 µM; n = 1.05; Mittel von 10 Experimenten.

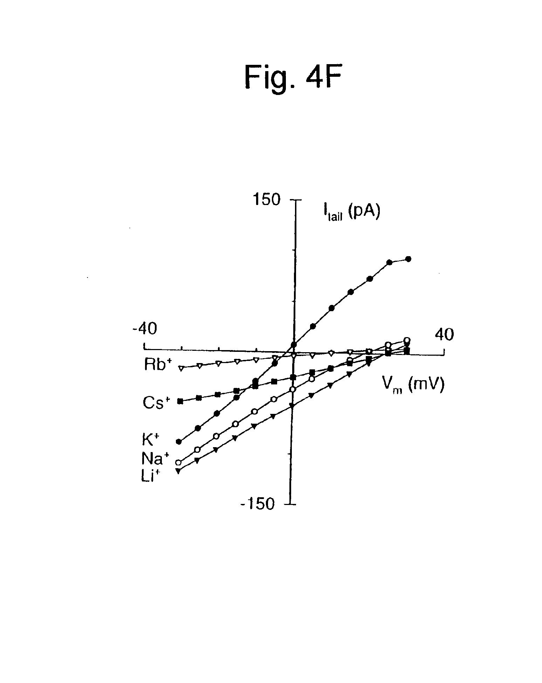

Die **Figur 4** zeigt mehrere pharmakologische Eigenschaften des SPIH-Kanals

Die **Figuren 4A und B** zeigen spannungsaktivierte SPIH-Ströme, aufgezeichnet durch Outside-Out-Membran-Patches ohne (A) und mit 10 mM Cs⁺ (B) im Bad; die Pipettenlösung enthielt das folgende (mM): 124 KCI, 10 Hepes-KOH, 10 EGTA bei pH 7,4 und 1 mM cAMP; die Badlösung enthielt (mM): 126 KCl, 10 Hepes-KOH, 10 EGTA bei pH 7,4 und die angezeigten Konzentrationen von CsCl.

**Figur 4C** zeigt wiederum die I/V-Beziehung in Gegenwart von 0 bis 10 mM Cs⁺ im Bad.

Aus **Figur 4D** ist die Abhängigkeit des normalisierten Stroms bei -70 mV auf [Cs⁺] ablesbar. Die durchgezogene Linie stellt eine Angleichung der Hill-Gleichung an diese Daten dar; Kᵢ = 245 µM, Hill-Koeffizient 1,2 (Mittel von 1-6 Experimenten).

**Figur 4E** zeigt die Ionenselektivität des SPIH-Kanals. Vᵣₑᵥ wurde auf Inside-Out-Patches durch Abstufung der Haltespannung (-70 mV) auf Testwerte zwischen -30 mV und +30 mV in 5 mV-Schritten bestimmt. Die Pipettenlösung enthielt das folgende (mM): 150 KCl, 10 Hepes-NMDG, 10 EGTA bei pH 7,4; die Badlösung war wie folgt zusammengesetzt (mM): 50 KCl, 100 XCl, 10 Hepes-NMDG, 10 EGTA bei pH 7,4 und 0,1 cAMP.

**Figur 4F** zeigt die I/V-Beziehung der Ströme, die im Teil E gezeigt werden. Vᵣₑᵥ betrug 16,9 mV (Na⁺), 20,6 mV (Li⁺), 5,6 mV (Rb⁺) und 24,6 mV (Cs⁺); Mittel von 3 bis 10 Experimenten. Die relativen lonenpermeabilitäten P_{X}/P_{K} wurden gemäß der Gleichung P_{X}/P_{K} = {[K⁺]ₒ - [K⁺]ᵢ exp(zFVᵣₑᵥ/RT)} /[X*]_{,} exp(zFVᵣₑᵥ/RT) berechnet.

**Figur 4G** zeigt die K⁺-Abhängigkeit von Gesamtzell- nach innen gerichteten Na⁺-Strömen in Gegenwart von 0.5 mM und 20 mM K⁺ im extrazellulären Medium

**Figur 4H** zeigt die "instantaneous" I/V-Beztehungen in Anwesenheit von 0, 1, 3. 5, 10. und 20 mM K⁺ im Bad.
Die Pipettenlösung war wie in Teil B, die Badlösung wie in Figur 1A mit den angezeigten K⁺-Konzentrationen; die Ionenstärken wurden auf den selben Wert durch die jeweiligen NMDG-Konzentrationen eingestellt.

**Figur 5** zeigt das Expressionsmuster von SPIH.

**Figur 5A** ist eine Northern-Blot-Analyse der Gewebeverteilung von SPIH-Transkripten in mRNA von männlichen Gonaden (Spur 1), weiblichen Gonaden (Spur 2) und Intestinalzellen (Spur 3); jeweils 10 µl Poly(A)⁺RNA.

**Figur 5B** ist eine Western-Blot-Analyse von Membranen von scheintransfizierten HEK293-Zellen (Spur 1; 2,5 µg Protein), HEK293-Zellen, die mit SPIH-cDNA transfiziert waren (Spur 2; 2,5 µg Protein), gereinigten Flagellen von Spermien von *S*. *purpuratus* (Spur 3; 6 µg Protein), dephosphorylierten Flagellenmembranen (Spur 4; 6 µg Protein) und von Spermienköpfen (Spur 5; 15 µg Protein).

### Referenztabelle der im Text durch SEQ ID Nummern beschriebenen DNA-Sequenzen

| SEQ ID NO | DNA Sequenz |
|---|---|
| 1 | Teilsequenz des Iₕ-Kanals aus menschlichem Thalamusgewebe |
| 2 | Teilsequenz eines Iₕ-Kanals aus olfaktorischem Gewebe der Ratte |
| 3 | Teilsequenz eines Iₕ-Kanals aus Retinagewebe vom Rind |
| 4 | vollständige Sequenz des Iₕ-Kanals aus Spermien des Seeigels |
| 5 | vollständige Sequenz des Iₕ-Kanals aus *Drosophila melanogaster* |
| 6 | Teilsequenz eines Iₕ-Kanals aus Retinagewebe vom Rind |
| 7 | Teilsequenz eines Iₕ Kanals aus Retinagewebe vom Rind |
| 8 | Teilsequenz eines lₕ-Kanals aus olfaktorischem Gewebe der Ratte |
| 9 | Teilsequenz eines Iₕ-Kanals aus olfaktorischem Gewebe der Ratte |
| 10 | Teilsequenz eines Iₕ Kanals aus menschlichem Thalamusgewebe |
| 11 | Teilsequenz eines Iₕ-Kanals aus menschlichem Herzgewebe |
| 12 | Vollständige Sequenz eines Iₕ-Kanals aus Retinagewebe vom Rind |
| 13 | Teilsequenz eines Iₕ-Kanals aus olfaktorischem Gewebe der Ratte |
| 14 | Teilsequenz eines Iₕ-Kanals aus olfaktorischem Gewebe der Ratte |
| 15 | Vollständige Sequenz eines Iₕ-Kanals aus menschlichem |
| | Herzgewebe |

### Literaturliste

Accili, E.A., Redaelli, G. and DiFrancesco, D.: Differential control of the hyperpolarization-activated current (i) by cAMP gating and phosphatase inhibition in rabbit sino-atrial node myocytes. J.Physiol. 500 (1997) 643-651.
Adams, S.R. and Tsien, R.Y.: Controlling cell chemistry with caged compounds. Annu. Rev. Physiol. 55 (1993) 755-784.
Aiba, H., Fujimoto, S. and Ozaki, N.: Molecular cloning and nucleotide sequencing of the gene for E. coli cAMP receptor protein. Nucleic Acids Res. 10 (1982) 1345-1361.
Altenhofen, W., Ludwig, J., Eismann, E., Kraus, W., Bönigk, W. and Kaupp, U.B.: Control of ligand specificity in cyclic nucleotide-gated channels from rod photoreceptors and olfactory epithelium. Proc.Natl.Acad.Sci.USA 88 (1991) 9868-9872.
Anderson, J.A., Huprikar, S.S., Kochian, L.V., Lucas, W.J. and Gaber, R.F.: Functional expression of a probable Arabidopsis thaliana potassium channel in Saccharomyces cerevisiae. Proc.Natl.Acad.Sci.USA 89 (1992) 3736-3740.
Araki, T., Ito, M. and Oshima, T.: Potential changes produced by application of current steps in motoneurones. Nature 191 (1962) 1104-1105.
Attwell, D. and Wilson, M.: Behavior of the rod network in the tiger salamander retina mediated by membrane properties of individual rods. J.Physiol. 309 (1980) 287-315.
Babcock, D.F., Bosma, M.M., Battaglia, D.E. and Darszon, A.: Early persistent activation of sperm K channels by the egg peptide speract. Proc.Natl.Acad.Sci.USA 89 (1992) 6001-6005.
Bader, C.R., MacLeish, P.R. and Schwartz, E.A.: A voltage-clamp study of the light response in solitary rods of the tiger salamander. J.Physiol. 296 (1979) 1-26.
Bader, C.R., Bertrand, D. and Schwartz, E.A.: Voltage-activated and calcium-activated currents studied in solitary rod inner segments from the salamander retina. J.Physiol. 331 (1982)253-284.
Baumann, A. Frings, S., Godde. M., Seifert, R. and Kaupp, U.B.: Primary structure and functional expression of a Drosophila cyclic nucleotide-gated channel present in eyes and antennae. EMBO J. 13 (1994) 5040-5050.
Beltrán, C., Zapata, O. and Darszon, A.: Membrane potential regulates sea urchin sperm adenylylcyclase, Biochemistry 35 (1996) 7591-7598.
Brown, H F and DiFrancesco. D Voltage clamp investigations of current underlying pacemaker activity in rabbit-sino-atrial note J Physiol 308 (1980) 221-251
Brown, H.F., DiFrancesco, D. and Noble, S.J.: How does adrenaline accelerate the heart? Nature 280 (1979) 235-236.
Darszon, A., Labarca, P., Beltrán, C., Garcia-Soto, J. and Liévano, A.: Sea urchin sperm: An ion channel reconstitution study case. Methods: A Companion to Methods in Enzymology 6 (1994) 37-50.
Darszon, A., Liévano, A. and Beltrán, C.: Ion channels: Key elements in gamete signaling. In Current Topics in Developmental Biology, Vol. 44. Academic Press, San Diego, 1996, pp. 117-167.
Dayhoff, M.O., Schwartz, R. M., Orcutt, B.C. (1978) in: Atlas of protein sequence and structure, Band 5, Suppl. 3, Hrsg.: Dayhoff, M.O., National Biomedical Research Foundation, Silver Spring, MD, S. 345-352.
DiFrancesco, D.: A new interpretation of the pace-maker current in calf Purkinje fibres. J. Physiol. 314 (1981 a) 359-376.
DiFrancesco, D.: A study of the ionic nature of the pace-maker current in calf Purkinje fibres. J. Physiol. 314 (1981b) 277-293.
DiFrancesco, D.: The hyperpolarization-activated current, i, and cardiac pacemaking. In Rosen, M.R., Janse, M.J. and Wit, A.L. (Eds.), Cardiac Electrophysiology: a Textbook. Futura, New York, 1990, pp. 117-132.
DiFrancesco, D.: Pacemaker mechanisms in cardiac tissue. Annu.Rev.Physiol. 55 (1993) 455-472.
DiFrancesco, D. and Tortora, P.: Direct activation of cardiac pacemaker channels by intracellular cyclic AMP. Nature 351 (1991) 145-147.
Fain, G.L., Quandt, F.N., Bastian, B.L. and Gerschenfeld, H.M.: Contribution of a caesium-sensitive conductance increase to the rod photoresponse. Nature 272 (1978) 467-469.
Finn, J.T., Grunwald, M.E. and Yau, K.-W.: Cyclic nucleotide-gated ion channels: An extended family with diverse functions. Annu.Rev.Physiol. 58 (1996) 395-426.
Garbers, D.L.: Molecular basis of fertilization. Annu. Rev. Biochem. 58 (1989) 719-742
Garbers, D.L.: Guanylyl cyclase receptors and their endocrine, paracrine, and autocrine ligands. Cell 71 (1992) 1-4.
Hagen, V., Dzeja, C., Frings, S., Bendig, J . Krause, E and Kaupp, U B.: Caged compounds of hydrolysis-resistant analogues of cAMP and cGMP Synthesis and application to cyclic nucleotide-gated channels. Biochemistry 35 (1996) 7762-7771
Halliwell, J.V. and Adams, P.R.: Voltage-clamp analysis of muscarinic excitation in hippocampal neurons. Brain Res. 250 (1982) 71-92.
Hansbrough, J.R., Kopf, G.S. and Garbers, D.L.: The stimulation of sperm metabolism by a factor associated with eggs and by 8-bromo-guanosine 3',5'-monophosphate. Biochim.Biophys.Acta 630 (1980) 82-91.
Hille, B.: lonic channels of excitable membranes. Sinauer Associates Inc., Sunderland, 1992.
Hodgkin, A.L. and Huxley, A.F.: A quantitative description of membrane current and its application to conduction and excitation in nerve. J. Physiol. 117 (1952) 500-544.
Ingram, S.L. and Williams, J.T.: Modulation of the hyperpolarization-activated current (I) by cyclic nucleotides in guinea-pig primary afferent neurons. J.Physiol. 492 (1996) 97-106.
Ito, M. and Oshima, T.: Electrical behavior of the motoneurone membrane during intracellularly applied current steps. J. Physiol. 180 (1965) 607-635.
Kaupp, U.B., Niidome, T., Tanabe, T., Terada, S., Bönigk, W., Stühmer, W., Cook, N.J., Kangawa, K., Matsuo, H., Hirose, T., Miyata, T. and Numa, S.: Primary structure and functional expression from complementary DNA of the rod photoreceptor cyclic GMP-gated channel. Nature 342 (1989) 762-766.
Labarca, P., Santi, C., Zapata, O., Morales, E., Beitrán, C., Liévano, A. and Darszon, A.: A cAMP regulated K-selective channel from the sea urchin sperm plasma membrane. Develop.Biol. 174 (1996) 271-280.
Lee, H.C. and Garbers, D.L.: Modulation of the voltage-sensitive Na/H exchange in sea urchin spermatozoa through membrane potential changes induced by the egg peptide speract. J. Biol. Chem. 261 (1986) 16026-16032.
Llinás, R.R.: The intrinsic electrophysiological properties of mammalian neurons: insights into central nervours system function. Science 242 (1988) 1654-1664.
Ludwig, J., Margalit, T., Eismann, E., Lancet, D. and Kaupp, U.B.: Primary structure of cAMP-gated channel from bovine olfactory epithelium, FEBS Lett. 270 (1990) 24-29
Pape, H.-C.: Queer current and pacemaker: The hyperpolarization-activated cation current in neurons. Annu.Rev.Physiol. 58 (1996) 299-327.
Pongs, O., Kecskemethy, N., Müller, R. Krah-Jentgens, I., Baumann, A. Kiltz, H.H., Canal, I., Llamazares, S. and Ferrus, A Shaker encodes a family of putative potassium channel proteins in the nervous system of Drosophila. EMBO J 7 (1988) 1087-1096.
Sanger, F., Nicklen, S. and Coulson, A.R.: DNA sequencing with chain-terminating inhibitors. Proc.Natl.Acad.Sci.USA 74 (1977) 5463-5467.
Smith, P.L., Baukrowitz, T. and Yellen, G.: The inward rectification mechanism of the HERG cardiac potassium channel. Nature 379 (1996) 833-836.
Suarez, S.S., Varosi, S.M. and Dai, X.: Intracellular calcium increases with hyperactivation in intact, moving hamster sperm and oscillates with the flagellar beat cycle. Proc.Natl.Acad.Sci.USA 90 (1993) 4660-4664.
Takio, K., Wade, R.D., Smith, S.B., Krebs, E.G., Walsh, K.A. and Titani, K.: Guanosine cyclic 3',5'-phosphate dependent protein kinase, a chimeric protein homologous with two separate protein families. Biochemistry 23 (1984) 4207-4218.
Titani, K., Sasagawa, T., Ericsson, L.H., Kumar, S., Smith, S.B., Krebs, E.G. and Walsh, K.A.: Amino acid sequence of the regulatory subunit of bovine type I adenosine cyclic 3',5'-phosphate dependent protein kinase. Biochemistry 23 (1984) 4193-4199.
Trudeau, M.C., Warmke, J.W., Ganetzky, B. and Robertson, G.A.: HERG, a human inward rectifier in the voltage-gated potassium channel family. Science 269 (1995) 92-95.
Varnum, M.D., Black, K.D. and Zagotta, W.N.: Molecular mechanism for ligand discrimination of cyclic nucleotide-gated channels. Neuron 15 (1995) 619-625.
Warmke, J., Drysdale, R. and Ganetzky, B.: A distinct potassium channel polypeptide encoded by the Drosophila eag locus. Science 252 (1991) 1560-1562.
Warmke, J.W. and Ganetzky, B.: A family of potassium channel genes related to eag in Drosophila and mammals. Proc.Natl.Acad.Sci.USA 91 (1994) 3438-3442.
Weiner, J.: Molekularbiologische, immunologische und funktionelle Charakterisierung von β-Untereinheiten des zyklisch Nukleotid-gesteuerten Ionenkanals aus dem Rinderhoden. Dissertation (1996) Universität Düsseldorf
Wollmuth, L.P. and Hille, B.: lonic selectivity of Ih channels of rod photoreceptors in tiger salamanders. J.Gen.Physiol. 100 (1992) 749-765.
Yanagihara, K. and Irisawa, H.: Inward current activated during hyperpolarization in the rabbit sino atrial node cell. Pflügers Arch 385 (1980) 11-19.

### SEQUENZPROTOKOLL

<110> Forschungszentrum Jülich GmbH
<120> Sequenzen eines Iₕ-Ionenkanals und deren Verwendung
<130> pct981
<140>
   <141>
<160> 17
<170> PatentIn Vers. 2.0

<210> 1
   <211> 1342
   <212> DNA
   <213> Homo sapiens

<400> 1
   cgttgcgctt caccaagatc ctcagcctcc tgcggctgct gcgcctctca cgcctgatcc 60
   gctacatcca tcagtgggag gagatcttcc acatgaccta tgacctggcc agcgcggtga 120
   tgaggatctg caatctcatc agcatgatgc tgctgctctg ccactgggac ggctgcctgc 180
   agttcctggt gcccatgctg caggacttcc cgcgcaactg ctgggtgtcc atcaatggca 240
   tggtgaacca ctcgtggagt gaactgtact ccttcgcact cttcaaggcc atgagccaca 300
   tgctgtgcat cgggtacggc cggcaggcgc ccgagagcat gacggacatc tggctgacca 360
   tgctcagcat gattgtgggt gccacctgct acgccatgtt catcggccac gccactgccc 420
   tcatccagtc gctggactcc tcgcggcgcc agtaccagga gaagtacaag caggtggagc 480
   agtacatgtc cttccacaag ctgccagctg acttccgcca gaagatccac gactactatg 540
   agcaccgtta ccagggcaag atgtttgacg aggacagcat cctgggcgag ctcaacgggc 600
   ccctgcggga ggagatcgtc aacttcaact gccggaagct ggtggcctcc atgccgctgt 660
   tcgccaacgc cgaccccaac ttcgtcacgg ccatgctgac caagctcaag ttcgaggtct 720
   tccagccggg tgactacatc atccgcgaag gcaccatcgg gaagaagatg tacttcatcc 780
   agcacggcgt ggtcagcgtg ctcactaagg gcaacaagga gatgaagctg tccgatggct 840
   cctacttcgg ggagatctgc ctgctcaccc ggggccgccg cacggcgagc gtgcgggctg 900
   acacctactg ccgcctctat tcgctgagcg tggacaactt caacgaggtg ctggaggagt 960
   accccatgat gcggcgcgcc ttcgagacgg tggccatcga ccgcctggac cgcatcggca 1020
   agaagaattc catcctcctg cacaaggtgc agcatgacct caactcgggc gtattcaaca 1080
   accaggagaa cgccatcatc caggagatcg tcaagtacga ccgcgagatg gtgcagcagg 1140
   ccgagctggg ctcagcgcgt gggcctcttc ccgccgccgc cgccgccgcc gcagtcacct 1200
   cggccatcgc cacgctgcag caggcggcgg ccatgagctt ctgcccgcag tggcgcggcc 1260
   gctcgtgggg ccgctggcgc tcggctcgcc gcgcctcgtg cgchgcyndy hcccggggsc 1320
   cgcacctgch gccncctcac cc                                         1342
<210> 2
   <211> 3112
   <212> DNA
   <213> Rattus rattus
<400> 2
   cctggttcgt ggtggacttc atctcctcga tcccggtgga ttatatcttt cttattgtag 60
   agaaaggaat ggattcggaa gtttacaaga ccgccagagc acttcggatc gtgaggttta 120
   caaaaattct cagtctcttg cgtttattac gcctttcaag gttaattaga tacatacacc 180
   agtgggaaga gatattccac atgacatatg atctcgccag tgcagtggtg agaatcttca 240
   acctcattgg catgatgctg ctcctgtgtc actgggatgg ctgtcttcag tttctggtcc 300
   ccctgctgca ggacttccca ccggattgct gggtttctct aaatgaaatg gttaatgatt 360
   catgggggaa acagtattcc tacgcactct tcaaagctat gagtcacatg ctgtgcattg 420
   gttatggcgc ccaggccccc gtcagcatgt ctgacctctg gattaccatg ctgagcatga 480
   ttgttggggc cacctgctat gccatgtttg tcggccatgc cacagctttg atccagtctc 540
   tggattcttc aaggaggcag tatcaagaga agtacaagca agtagagcaa tacatgtcat 600
   tccacaagtt accagctgac atgcgccaga agatacatga ttactatgag caccgatacc 660
   aaggcaagat cttcgatgag gaaaatattc tcagtgaact taatgatcct ctgagagagg 720
   aaatagtcaa cttcaactgc cggaaactgg tggccaccat gcctctcttt gctaacgcgg 780
   atcccaattt cgtgacggcc atgctgagca agctgagatt tgaggtgttc cagcccggag 840
   actatatcat tcgagaagga gctgtgggga agaaaatgta tttcatccag catggtgtgg 900
   ctggtgtcat caccaagtcc agtaaagaaa tgaagttgac agacggctct tactttggag 960
   aaatatgcct gctgaccaag ggccggcgca ctgccagtgt tcgagctgat acatactgtc 1020
   gcctttactc cctttcggtg gacaatttca acgaggtctt ggaggaatat ccaatgatga 1080
   gaagagcctt tgagacagtt gctattgacc gactagatcg gataggcaag aaaaactcta 1140
   ttctcctgca gaagttccag aaggatctga acactggtgt tttcaacaac caggagaatg 1200
   agatcctgaa gcagattgtg aagcatgaca gagagatggt acaagcgatc cctccaatca 1260
   actatcctca aatgacagcc ctgaattgca catcttcaac caccacccca acgtcgcgca 1320
   tgaggaccca atctccacca gtctacacag cgaccagcct ctctcacagc aacctgcact 1380
   cacccagccc cagcacacag acgcctcaac cctcagccat cctttcaccc tgctcctaca 1440
   ccacagcagt ctgcagtcct cctatacaga gccccctggc cacgcgaact ttccattatg 1500
   cctctcccac tgcatcccaa ttgtcactca tgcagcagcc tcagccgcag ctacagcaat 1560
   cccaggtaca gcagactcag ccgcagccgc agccgcagcc gcagcagccg caacagcaac 1620
   aacagcagca acagcagcag cagcagcagc agcaacaaca acagcagcag caacagccac 1680
   agacacctgg tagttccaca ccgaaaaatg aagtgcacaa gagcactcaa gctcttcata 1740
   acaccaacct gaccagagaa gtcaggcccc tctctgcctc gcagccttcg ctgccccatg 1800
   aggtctccac tatgatctcc agaccgcatc ccactgtggg cgagtccctg gcctccatcc 1860
   ctcaacccgt ggcaacagtc cacagcactg gccttcaggc agggagcagg agcaccgtgc 1920
   cacagcgtgt caccttgttc agacagatgt cctcgggagc tatttccccc aaccgaggag 1980
   tgcctccagc acccccacca ccagcagctg tgcagagaga gtctccctca gtcttaaata 2040
   aagacccaga tgcagaaaaa ccacgttttg cttcgaattt atgattcttg ctgattgtca 2100
   aagcagaaaa gaaatactct aataaacaga atattctcag atattatttt attctatctc 2160
   atgatagagc cctatagcct actctaaaaa gatattttag aagctctggc gtacatgcaa 2220
   atgtaaaaac atatatacat atattattaa atatatatat atatctaaat gcccaagaga 2280
   agttcaaaag acttgtataa ctttcagtgt tatgtcttcc tttctttaaa accattaaag 2340
   gatttaacac attgttgtaa gatcattgat ttctaacctt ttacttaatt cctttgttat 2400
   atgtgtttct cccttttatg aagagttctt gaagtcattg gaaacaaaac tctgatttag 2460
   aaataaaagg caactccaat tagtttcagc atagcaccaa tcaaagcttt ctttcattaa 2520
   ctgtgcctct gcatctaggt tgttaattat gtgggattca ataaagaaat cccagtttat 2580
   agctctaaat tgtattttgg tgctttaaat tttgagttat gtgaaggaac acactacacg 2640
   ctcagccacc ataggagact aacattgcca ctgttaaggc ttcctctaac ctcaaacatg 2700
   ttcgtcaatt ttgtgaggaa aggtgaggag atatttgtct tcatgtgtta ttggactttt 2760
   accaagattc agtcaatgtt agctgtaaat aacttttcca acctgaataa aagtaactat 2820
   tctgtgttgt ataaaggtaa aagtcactgt ttaagaattt agttttattg cttcacttca 2880
   aaagttagag ttttaaaatt tcacaaaaca taataattgt gacaactgtt caaatgtaat 2940
   gcaattgctt gagacctaca atatcattta aacctgcaat attttatgca aaaattgtat 3000
   gcttgaacct acaaattgct tgtattacac caaaaatcat tacttttatt ccttcttgac 3060
   ataatcaagc atctgaacct agtcctggca tgcttttggg ggcaaaaaaa aa 3112
<210> 3
   <211> 2606
   <212> DNA
   <213> Bos taurus
<400> 3
   cgggagcccg gagcgcagcc actgagggca gcggcggcgg cgggagcgag gcgcgcagcg 60
   agaagcggcg gcgaggaatc ggccgggggc ttcgaggacg ccgaggggcc ccggcggcag 120
   tacggcttca tgcagcggca gttcacctcc atgctgcagc ccggggtcaa caaattctcc 180
   ctccgcatgt tcgggagcca gaaggcggtg gagaaggagc aggaaagggt taaaactgca 240
   ggcttctgga ttatccaccc ttacagtgat ttcaggtttt attgggattt aataatgctt 300
   ataatgatgg ttggaaatct ggtcatcata ccagttggaa tcacattctt tacagaacag 360
   acaacaacac catggattat tttcaatgtg gcttcagata cagttttcct tttggacttg 420
   atcatgaatt tcaggactgg gactgtcaat gaagacagtt ctgaaatcat cctggaccct 480
   aaagtgatca agatgaatta tttaaaaagc tggtttgtgg ttgacttcat ctcatcaatc 540
   ccagtggatt atatctttct cattgtagaa aaaggaatgg attcggaagt ttacaagaca 600
   gccagggcac ttcgcattgt gaggtttaca aaaattctca gtctcttgcg tttattacga 660
   ctttcaaggt taattagata catacatcag tgggaagaga ttttccacat gacatatgat 720
   cttgccagtg ctgtggtgag aatttttaac ctcattggca tgatgctgct cctgtgccac 780
   tgggatggct gtcttcagtt cctggtacca ctgctgcagg acttcccacc agattgctgg 840
   gtgtctctaa atgagatggt taatgattct tggggaaagc agtattccta cgcgctcttc 900
   aaagcgatga gtcatatgct gtgcattggc tacggagccc aagcccccgt gagcatgtct 960
   gacctgtgga tcaccatgct gagcatgatc gtcggggcca cctgctacgc catgtttgtt 1020
   ggccacgcca cggctctaat tcagtctttg gattcctcaa ggcggcaata tcaagagaag 1080
   tataagcaag tggaacaata catgtcattc cataagttac cagctgatat gcgtcagaag 1140
   atacatgatt attatgaaca cagataccaa ggcaaaatct ttgatgagga aaatattctc 1200
   aatgaactca atgatcctct gagagaggag atagtcaact tcaactgccg aaaactagtg 1260
   gctacaatgc ctctttttgc taatgcggat cctaatttcg tgaccgccat gctgagcaag 1320
   ttgagatttg aggtgtttca acctggagat tatatcatac gagaaggagc tgtggctaaa 1380
   aaaatgtatt tcattcaaca tggtgttgct ggtgtcatca caaaatccag taaagaaatg 1440
   aagctgacag atggctcata ctttggagag atttgcttgc tgaccaaggg acggcgcact 1500
   gccagtgttc gagctgatac atattgtcgt ctttactcac tttctgtgga caatttcaat 1560
   gaggtcctgg aggaatatcc aatgatgaga agagcctttg agacggttgc cattgaccga 1620
   ttagatagga tagggaagaa aaattcaatt ctcctgcaaa agttccagaa ggatctgaac 1680
   acgggtgttt tcaacaatca ggagaacgag atcctgaagc agattgtgaa acacgacagg 1740
   gaaatggtgc aggcaatccc tcccctcaat taccctcaaa tgacagccct gaattccacc 1800
   tcttcaacta ctaccccgac ctctcgcctg aggacacagt caccgccagt gtacacagcc 1860
   accagtctgt ctcatagcaa cctgcactcc cccagcccca gcacccagac cccccagccg 1920
   tcagccatcc tctcgccctg ctcctacacc accgctgtct gcagccctcc tgtacagagc 1980
   ccgctagcca ctcgaacttt ccactatgcc tcccccacgg cttcccagtt gtccctcatt 2040
   cagcagcagc aggttcagca gccaccgcag ccccagcagc caccccaacc tccacagacc 2100
   cccggcagct ccacaccgaa aaacgaagtg cacaagagca cgcaggcgct tcacaacacc 2160
   agcctgaccc gagaagtcag gcccctctcg gcctcgcagc cctcgctgcc ccacgaggtc 2220
   tccaccctga tctccagacc gcatcccact gtgggcgagt ccctggcctc catccctcaa 2280
   cccgtgacca cggtccacgg ctcgggcctg caggcagggg gcaggggcac cgtcccccag 2340
   cgagtcaccc tgttccgaca gatgtcatcg ggagccatcc cccccaatcg aggagtcccc 2400
   ccggcccccc ctccaccagc agccgctcat ccgagggagg cgccctcagt cttaactaca 2460
   gactcagagg cagaaaagcc acgatttgct tcaaatttat gatcctgctg attgtaaagc 2520
   agaaagaaat actctaacgt aactgaggac gcttctcaga tttgatttta ttctatctcc 2580
   tgatagatcc tctagcctac tatgaa                                      2606
<210> 4
   <211> 2935
   <212> DNA
   <213> Strongylocentrotus purpuratus
<400> 4
   atcaaacccg ggccccatca tgaaggaata acaaggcctt cgaaaagtat gggaaactgg 60
   tcggcaggac atcagcatta ttaattctag gaaactcatt atggataaca aggaaactaa 120
   cggagagcta gagcagtctg atgaggccga tccgtccggt caaaaccttg atgatgggga 180
   aaccgatagc aaacaagaag agaatctcat caacgttagc ccgccaaaaa caccgccagg 240
   tcctcctcct cctctaaaga atggaggaag gggtcagaaa ccgcccaaaa tcccaatatg 300
   tcatcaaaat ggaaagctcc ccaaggaagt tgaatggaca gaagacagag gcgaagacag 360
   aaaggatagt ctcactcttc aatcaaagct agatcacggg gcatacacgg atgagaaaca 420
   ggatcttcta acatatcttg accgtcacgg catcaacagt ccagtcaagc taacaccaga 480
   tgaaactgga gggagcagtg ctttggatat tcttgggatt attgaagaga gggacactgg 540
   tgcactaggc tctgatccct catccactat gcaggccatg gctaaacctg taggctttct 600
   gcagaggcag ctatggactg tcctccaacc ttcagacaat agactctcca tgaaactttt 660
   cggaagcaag aaagggttac aaaaggaaaa atatcggctg aggaaggcgg gggttcttat 720
   cattcatcca tgtagtcatt tcagatttta ctgggatcta ctgatgctgt gcctgatcat 780
   ggcaaacgtc atcctcctac ccgtcgtcat tactttcttc cacaacaagg acatgagtac 840
   gggttggctc atctttaatt gcttctcaga taccttcttc attctcgatc tcatctgcaa 900
   ctttcggacc ggcatcatga atccgaagtc ggccgaacag gtgatcctca acccccgtca 960
   aatcgcctat cattatctcc gttcatggtt catcatcgat ctcgtgtctt ccatccccat 1020
   ggactacatc ttcctcctcg ctggcggcca gaaccgtcac ttcctcgagg tgtcccgagc 1080
   cctcaagata ctgcgctttg ccaagctcct cagtcttctt cgactcctgc gtctgtccag 1140
   gctcatgcgg ttcgtcagtc aatgggaaca ggccttcaac gtagccaatg ccgtcatccg 1200
   gatctgtaat ctagtgtgta tgatgcttct gattggccat tggaatggct gccttcaata 1260
   tctcgtgccc atgctgcaag aataccccga ccaatcatgg gtcgccatta atggccttga 1320
   gcacgctcat tggtgggagc agtatacatg ggcactcttc aaagcccttt cgcacatgct 1380
   ctgtatcggg tacggcaagt tcccccctca aagcatcacc gatgtctggc taacgattgt 1440
   cagtatggtg tccggtgcga cctgcttcgc cctgttcatc ggacacgcta ccaatctcat 1500
   ccagtccatg gactcctcca gcaggcaata ccgtgagaag ttgaaacaag ttgaagagta 1560
   catgcagtat cgcaagctac cgtcccacct acgaaacaag atcctcgatt actacgagta 1620
   ccgataccga ggaaagatgt ttgatgagag gcatatcttt cgagaagtgt cggagagtat 1680
   acgacaggat gtcgcaaact acaattgtcg cgacctggtc gcatccgtcc ctttcttcgt 1740
   cggtgccgac tcaaacttcg tcacccgtgt ggtgacgctg ctcgaattcg aggtcttcca 1800
   acccgctgac tatgttatac aggaaggtac tttcggtgat cgcatgttct tcatccagca 1860
   gggcatcgtc gacatcatca tgtccgacgg cgtcatcgcc acgtcactca gtgacggctc 1920
   atattttggc gaaatctgcc tgcttacccg tgagcgccgc gtggcatcgg tgaagtgcga 1980
   gacctactgc acgctcttct cgctctccgt ccagcatttc aaccaagtgc tcgacgagtt 2040
   tcccgccatg aggaaaacga tggaagagat agccgttcgt cgtctgaccc gaatcgggaa 2100
   ggaatcgagc aagctgaaat cccgcctaga gagcccgacg atcagggaca ctgcccctct 2160
   ctttccgatc ccacctgata caccgtcttt cgtcaccgac atcgaaaaga accggttctt 2220
   tggcgacgac acggacgatg tacacatcag gacccgagtc gacgtcgagc gtggttcgca 2280
   tgaaaacgtc atcgccatca tggatgggag tttatccgac ctcaggatgg aaaacgaaat 2340
   ccaagcccgt aaatcgtcta gcggaaaacg gaggaaattc cagcaacaaa caaccgaact 2400
   atgacgactt gaaacaaaca atgatggacg cttacaattt ccagtgattc aatacttacg 2460
   caatgcagac attagctttt gtacctgatt gtttagaatg tattgaattt gtagatcagt 2520
   ccggcaaata agaaagcata atttggaatt tctttcattg aggaagtact gaaaacaatg 2580
   tgatagcagc cggtagaaat ttcttgtcca ttatcgaggc tatatttttc gcgctttctt 2640
   acgaagtaaa tgaaaggatc aattaaatta ttgttctttg tctsstgcgc tttgtatctg 2700
   atgccgaaaa ggaatgaaac gtgattagaa cagtaatcga ttgaactaca gaagtctttt 2760
   caaaatgttg aatgtatgaa ggaggagggg gaaggtttga tatatgcaaa gaaatggaga 2820
   aatatttttg taaatttatc tagaatggta ctattgatgc tggaaaggtg ttgaagttgt 2880
   ccaatattgt gtcaaatcac caactatttg acatttgtct ttttcccgga attcc      2935
<210> 5
   <211> 3185
   <212> DNA
   <213> Drosophila melanogaster
<400> 5
   cggaatttcc tcgctgaagg gcaaggggca gagtcagagt caggggcaga gcggcagacg 60
   ctgcccggcc atcgcgggtc ggtgaggagc gagagtggaa gcgggagcag ccacaccatt 120
   ccggcgacgg gcaagagtcc gccggtgccg cactcgctgg cggccaagat cagcagctcg 180
   gcaagcggca gcaagaactg caatttgctc agcgccagca gcaactcatg ccacaagctg 240
   aacgcccacg cccaaggatc ggagcaggat cgggatcttg gatcgggatc aggatcagga 300
   ccacccggac acagtcacta cgcggccgcc tcgcccaaaa gctcggtcag cagcaacggt 360
   catctgaaca agtactgcct cacggacctc acgcgccgca acgcgagttc aatcgccagc 420
   tgagcgcgcc cacggactac acgcaccact cctccagcaa cggatcgcag caggagggct 480
   cctcggaggc caacgagggc cacgaaccgg tcggcgagtc caccatcacc gtagccagtg 540
   ccggcgtatc gtatccgcat ccgtactcct atccgtatca ttacggcacc accgctcctc 600
   ggccacagcg ccggccaatc tcaaggcgtc gctgcagctg cacagctttg ggagccacca 660
   tccgtgtcct tatccggcaa ggcccacgtc cacgtcgtgc accaacagct tcaaccggcg 720
   ccacattcgc cggcacaagg gcaagctcgg cgatcgactg ctgagcgggg atagtgagga 780
   atcggtgcgc tgctcctatt gctcggtgct gaatgcgaac gacaacgacc tgcgcatttc 840
   gttcgagaac acctgcaccg attcgctggt aaccgctttc gatgatgaag ccctgctaat 900
   atgcgaccaa ggaaccgaaa tggtacactt tgatgacgtg tcgttgtacg gcactccgaa 960
   agaggagccc atgcccaaca taccgatcgt gtcggaaaaa gtctctgcga atttcctaaa 1020
   aagtcaattg caatcatggt tccagccgac ggacaaccga ctggccatga aactgtttgg 1080
   cagccgaaag gcgctggtca aggagcgcat acgtcagaaa acttccgggc actgggtcat 1140
   acacccgtgc agttcattca ggttttactg ggacctttgc atgcttttat tattagtagc 1200
   aaatcttatt atcctgccag tcgcaatatc attcttcaac gatgatctga gcacacgatg 1260
   gattgccttc aactgcctaa gtgatactat ttttttaata gatattgtag tcaattttag 1320
   aacaggaatt atgcaacaag acaacgctga acaagtaata ttggatccaa agcttatagc 1380
   taaacactat ttaagaactt ggttttttct cgatttgatt tcgtcgatac cgctagatta 1440
   tatattttta attttcaatc aaattatgaa attgcaggat ttctctgatt cttttcaaat 1500
   attgcatgcc ggacgcgccc tgccgatcct gcgcctggcc aagctgttat ccctggtgcg 1560
   actgctccgc ctttcccgcc tcgtccgcta cgtttcccaa tgggaggagg tctatttcct 1620
   caatatggcc tcggtcttca tgaggatctt caatttaatt tgcatgatgc tcctgatcgg 1680
   ccattggagc ggttgcttgc agttcttagt gccaatgttg cagggttttc catccaactc 1740
   ctgggtctcc atcaacgagt tgcaggaatc gtactggctg gagcagtatt cgtgggcatt 1800
   gttcaaggcc atgtcgcaca tgctctgcat aggctacggc agattcccgc cacaatcact 1860
   gacagacatg tggctgacga tgctatcgat gatatccggg gccacctgtt acgcattgtt 1920
   cctcggtcac gcgaccaatc tcatccagag cttggactcc agccggcgcc agtatcgcga 1980
   gaaggtcaaa caggtggagg agtacatggc ctaccgcaag ctgccacgcg acatgcggca 2040
   gcgcatcacg gaatatttcg agcatcggta ccagggtaaa ttcttcgatg aagagttgat 2100
   acttggcgag ttgagcgaaa aactgcgcga ggatgtcatc aactacaact gcagatccct 2160
   cgtggcgtca gtgccttttt ttgctaatgc cgattcgaat ttcgtttccg acgtagttac 2220
   caaactgaaa tacgaagttt tccaaccagg tgatattatc ataaaggagg gtacgatcgg 2280
   tactaagatg tacttcatac aggagggcgt ggtggacatt gtcatggcca acggcgaggt 2340
   tgccacctca ctttcggatg ggtcttattt cggtgagatc tgtctgctga ccaatgcgcg 2400
   tcgtgtggcc agcgtgcgag ccgaaaccta ttgcagtcta ttctcgttga gcgtggatca 2460
   tttcaattgc gttctggatc agtatccgct gatgcgcaag accatggaga ctgtggccgc 2520
   cgagcggtta aacaagatcg gcaagaatcc aaacataatg catcagaagg acgagcagct 2580
   gagcaatccg gagtcgaaca cgattacggc tgtggttaat gcactggctg ccgaggcgga 2640
   tgactgcaaa gatgatgaca tggatctcag ggagaattta ctgcatgggt cagagtcgag 2700
   cattgctgag ccggtgcaga cgatacgtga gggtctcccg aggccacgga gcggggagtt 2760
   ccgggccttg ttcgagggta acactccatg acactgagga gcagtgacaa gcggtgccct 2820
   cgggcaccgg gcaaccatct gaagcagcag ttcgctggac actcactcac caagtcccac 2880
   atccatactc cacacaggac taccactcac acacacacac acactgcgta tataataatt 2940
   tagtaaaagg aaccccaaga cgcgataaga gtacactaaa aaaagaatca atttatggta 3000
   gacactctat atatgcaatt gcgatttagt agaaaacgta ttaaaaacta aaaacccaaa 3060
   aaaagaagat aaaaacaatt acacaaaaaa tgtcctcaat aattattcat aatttcagct 3120
   ccgctaactg tgatgacttt aatataagaa tcgaaaaaaa aattaacaaa caaacaaaaa 3180
   aaaag                                                             3185
<210> 6
   <211> 2922
   <212> DNA
   <213> Bos taurus
<400> 6
   cgggagcccg gagcgcagcc actgagggca gcggcggcgg cgggagcgag gcgcgcagcg 60
   agaagcggcg gcgaggaatc ggccgggggc ttcgaggacg ccgaggggcc ccggcggcag 120
   tacggcttca tgcagcggca gttcacctcc atgctgcagc ccggggtcaa caaattctcc 180
   ctccgcatgt tcgggagcca gaaggcggtg gagaaggagc aggaaagggt taaaactgca 240
   ggcttctgga ttatccaccc ttacagtgat ttcaggtttt attgggattt aataatgctt 300
   ataatgatgg ttggaaatct ggtcatcata ccagttggaa tcacattctt tacagaacag 360
   acaacaacac catggattat tttcaatgtg gcttcagata cagttttcct tttggacttg 420
   atcatgaatt tcaggactgg gactgtcaat gaagacagtt ctgaaatcat cctggacccc 480
   aaagtgatca agatgaatta tttaaaaagc tggtttgtgg ttgacttcat ctcatcaatc 540
   ccagtggatt atatctttct cattgtagaa aaaggaatgg attcggaagt ttacaagaca 600
   gccagggcac ttcgcattgt gaggtttaca aaaattctca gtctcttgcg tttattacga 660
   ctttcaaggt taattagata catacatcag tgggaagaga ttttccacat gacatatgat 720
   cttgccagtg ctgtggtgag aatttttaac ctcattggca tgatgctgct cctgtgccac 780
   tgggatggct gtcttcagtt cctggtacca ctgctgcagg acttcccacc agattgctgg 840
   gtgtctctaa atgagatggt taatgattct tggggaaagc agtattccta cgcgctcttc 900
   aaagcgatga gtcatatgct gtgcattggc tacggagccc aagcccccgt gagcatgtct 960
   gacctgtgga tcaccatgct gagcatgatc gtcggggcca cctgctacgc catgtttgtt 1020
   ggccacgcca cggctctaat tcagtctttg gattcctcaa ggcggcaata tcaagagaag 1080
   tataagcaag tggaacaata catgtcattc cataagttac cagctgatat gcgtcagaag 1140
   atacatgatt attatgaaca cagataccaa ggcaaaatct ttgatgagga aaatattctc 1200
   aatgaactca atgatcctct gagagaggag atagtcaact tcaactgccg aaaactagtg 1260
   gctacaatgc ctctttttgc taatgcggat cctaatttcg tgaccgccat gctgagcaag 1320
   ttgagatttg aggtgtttca acctggagat tatatcatac gagaaggagg ctgtggtaaa 1380
   aaaatgtatt tcattcaaca tggtgttgct ggtgtcatca caaaatccag taaagaaatg 1440
   aagctgacag atggctcata ctttggagag atttgcttgc tgaccaaggg acggcgcact 1500
   gccagtgttc gagctgatac atattgtcgt ctttactcac tttctgtgga caatttcaat 1560
   gaggtcctgg aggaatatcc aatgatgaga agagcctttg agacggttgc cattgaccga 1620
   ttagatagga taggtactgt ttattttctt ctttacttac aattcacttt taatctagtg 1680
   gttgagtata tatttgcagt cataagtccc aaatgctagt ttacagattg cttattaact 1740
   agcatagaaa cagcaattag ctgtagccat atttctagaa gatctgaggc actaacttct 1800
   cgtctaagta ttctaggttt gtttattcat ctctgttttt actagcttca cagtctgatt 1860
   tcctcagtga taccaaaagg taaaaccaat gattacaaat tctagatggc attaaaatag 1920
   wwctnaaaaa tacaatagta tgagtctaca ttacaaacta tattttatna caagtttttt 1980
   ttttaanntt aagggtcaac attacattta ttcttatatt aagaattgaa aagaattgtg 2040
   cattttactt gtcacagtag aaacgttaat gtttgtaata crrrctcaag cagaaaaagc 2100
   cttaatagaa ctgcccacat agatgcttta ttttgcaaac atcaacttat tttaaaatct 2160
   ttcctgctct caaattaaaa tattgatata taaggcctta ctagttatac tagtttaaac 2220
   gtctgaataa ttgccatgta aaaattagat cagattggct tgctgttaac ttcccaagat 2280
   atgctggaac attctgatgt cagaaggtgg tatgcattca ttttccacac ccaaattctc 2340
   ctccccgacc agacccttct ctgctccctt tcccagctta actctactag ccttcatagt 2400
   tcaatttaaa catcatttcc ctgtagaaac cnatngacct tccactcctc cttaatrrta 2460
   tgagcaccct ggatatgttc tnccataccc ctgggatgtt cctccatcac agtacagntt 2520
   ttattattta aattgctcta gagatnctaa gctttatgaa tnaagagatc angtctaatt 2580
   cactattaca ttcacagtac cwwgtacaca atgaatattg ttgaagagag ttagggaggg 2640
   atgaaggaat caatgaactc aaaggagatg gggttgggat cactgaaaag taaacaaaga 2700
   ggtacttcaa ctgcttcatt cttattaaag gtaaggactt ttgattgatg ttacanttat 2760
   gttagctttt cttctgcact ttancatctt tcttttcctc tatattagta ggacagaaga 2820
   ctgcataagg atctagggtt tggttaggac aagtaaaggt agtatttggg cattaccatt 2880
   atggacacaa caaggcttcc aggtggataa caataataac gg 2922
<210> 7
   <211> 1820
   <212> DNA
   <213> Bos taurus
<400> 7
   cggcggacga ggcgggcagc gaggaggcgg gcccggcggg ggagtcgcgc ggcagccagg 60
   ccagcttcat gcagcgccag ttcggcgcgc tcctgcagcc gggcgtcaac aagttctcgt 120
   tgcggatgtt cggcagtcag aaggccgtgg agcgcgacga ggagcgcgtt aagtcagcgg 180
   gggcctggat catccaccct tacagcgact tcaggttcta ctgggacttc accatgctgc 240
   tcttcatggt gggaaacctc atcatcatcc ccgtgggcat caccttcttc aaggacgaga 300
   ccacggcccc atggattgtg ttcaatgttg tctcggacac attcttcctc atggacctgg 360
   tgctgaactt ccgcacgggc attgtgatcg aggacaacac ggagatcatc ctggaccccg 420
   agaagatcaa gaagaagtac ctgcgcacgt ggttcgtggt ggacttcgta tcctccatcc 480
   ccgtggtaag ctacatcttc ctcatcgtgg agaaaggcat cgactctgag gtctacaaga 540
   cggcccgcgc cctgccgatc gagccgttca ccaagatcct cagcctgctg cgcctgctcc 600
   gcttgtcgcg cctcatccgc tacatccatc agtgggagga gatcttccac atgacctacg 660
   acctggcgag cgccgtcatg cgcatctgca acctcatcag catgatgctg ctcctctgcc 720
   actgggatgg ctgcctgcag ttcctggtgc ccatgcttca ggacttccca cgcaactgct 780
   gggtctccat caacggcatg gtgaaccact catggagcga gctctactcc ttcgcgctgt 840
   tcaaggccat gagccacatg ctgtgcatcg ggtacgggcg gcaggcgcca gaaagcatga 900
   cggacatctg gctgaccatg ctgagcatga tcgtgggtgc cacctgctac gccatgttca 960
   ttggccacgc caccgccctc atccagtcgc tggactcctc aaggcgccag taccaggaga 1020
   agtacaagca agtggagcag tacatgtcct tccacaagct gccagccgac ttccgccaga 1080
   agatccacga ctactacgag caccgctacc agggcaagat gttcgacgag gacagcatcc 1140
   tcggcgagct caaggcgggc ctgcgggagg agatcgtcaa cttcaactgc cggaagctgg 1200
   tggcctccat gccactgttc gccaatgctg accccaactt cgtcacgggc catctgacca 1260
   agctcaagtt tgaggtcttc cagccaggcg actacatcat ccgtgagggc accattggca 1320
   agaagatgta cttcatccaa cacggcgtgg tcagtgtgct taccttgggc aacaaggaga 1380
   tgaagttgtc tgatggctcc tactttgggg agatctgcct gctgacgcgg ggccggcgca 1440
   cggcgagcgt ccgggccgac acctactgcc gcctctactc gctgagtgtg gacaacttca 1500
   atgaggtgct ggaggagtac cccatgatga ggcgggcctt tgagacagtc gccattgacc 1560
   gcctggatcg cattggcaag aagaactcga tcctgctaca caaggtgcag cacgacctca 1620
   actctggcgt gtttaacaac caggagaacg ccatcatcca ggagattgtc aagtatgacc 1680
   gcgagatggt gcagcaggct gagctgggcc agcgtgtcgg cctcttcccg ccaccaccgc 1740
   cacctccaca gggcacctca gccattgcca cgctgcagca gccgtggcca tgagcttctg 1800
   tccacaagtc gcacgccccc 1820
<210> 8
   <211> 101
   <212> DNA
   <213> Rattus rattus
<400> 8
   ctacatcatc cgagagggga ccatcgggaa gaagatgtac ttcatccagc acggggtggt 60
   gagcgtgcta accaggggca acaaggagga taagctgtca n 101
<210> 9
   <211> 558
   <212> DNA
   <213> Rattus rattus
<400> 9
   tctggtggtg cgtgagggct ccgtgggcag gaagatgtac ttcatccagc atggcgtgct 60
   cagtgtgttg gcacggggcg ctcgggacac tcgcctcact gacggatcct actttgggga 120
   gatctgcctg ctgactcgag gtcggagaac agccagtgta agggctgaca cctactgtcg 180
   cctctactca ctcagcgtgg accacttcaa tgcagtgctt gaggagctcc cgatgatgcg 240
   cagggctttt gagactgtgg ccatggaccg gcttcggcgc atcggtgagg cctgtctgcc 300
   ctgtctgctc tgggccctgc ctgagcctca tctcattttc atagcaagga acctacccct 360
   agtgtttctt ctccacaccc caacctaccc agtaccagca ggctattagc tctgtttctc 420
   gctagtctta cccctagaaa gaaatagcca tggagctgtc tccccaaacc ctcattccct 480
   gtgtcctctc gggtaccagt acttaacctc accgtttttg ataccacctt ccagtttctg 540
   ttgccaagca ttctctcc 558
<210> 10
   <211> 2886
   <212> DNA
   <213> Homo sapiens
<400> 10
   gaattcgcgg ccgcgtcgac ggccagcttc atgcagcgcc agttcggcgc gctcctgcag 60
   ccgggcgtca acaagttctc gctgcggatg ttcggcagcc agaaggccgt ggagcgcgag 120
   caggagcgcg tcaagtcggc gggggcctgg atcatccacc cgtacagcga cttcaggttc 180
   tactgggact tcaccatgct gctgttcatg gtgggaaacc tcatcatcat cccagtgggc 240
   atcaccttct tcaaggatga gaccactgcc ccgtggatcg tgttcaacgt ggtctcggac 300
   accttcttcc tcatggacct ggtgttgaac ttccgcaccg gcattgtgat cgaggacaac 360
   acggagatca tcctggaccc cgagaagatc aagaagaagt atctgcgcac gtggttcgtg 420
   gtggacttcg tgtcctccat ccccgtggac tacatcttcc ttattgtgga gaagggcatt 480
   gactccgagg tctacaagac ggcacgcgcc ctgcgcatcg tgcgcttcac caagatcctc 540
   agcctcctgc ggctgctgcg cctctcacgc ctgatccgct acatccatca gtgggaggag 600
   atcttccaca tgacctatga cctggccagc gcggtgatga ggatctgcaa tctcatcagc 660
   atgatgctgc tgctctgcca ctgggacggc tgcctgcagt tcctggtgcc tatgctgcag 720
   gacttcccgc gcaactgctg ggtgtccatc aatggcatgg tgaaccactc gtggagtgaa 780
   ctgtactcct tcgcactctt caaggccatg agccacatgc tgtgcatcgg gtacggccgg 840
   caggcgcccg agagcatgac ggacatctgg ctgaccatgc tcagcatgat tgtgggtgcc 900
   acctcgtacg ccatgttcat cggccacgcc actgccctca tccagtcgct ggactcctcg 960
   cggcgccagt accaggagaa gtacaagcag gtggaacagt acatgtcctt ccacaagctg 1020
   ccagctgact tccgccagaa gatccacgac tactatgagc accgttacca gggcaagatg 1080
   tttgacgagg acagcatcct gggcgagctc aacgggccnn tgcggnagga gatcgtcaac 1140
   ttcaactgcc ggaagctggt ggcctccatg ccgctgttcg ccaacgccga ccccaacttc 1200
   gtcacggcca tgctgaccaa gctcaagttc gaggtcttcc agccgggtga ctacatcatc 1260
   cgcgaaggca ccatcgggaa gaagatgtac ttcatccagc acggcgtggt cagcgtgctc 1320
   actaagggca acaaggagat gaagctgtcc gatggctcct acttcgggga gatctgcctg 1380
   ctcacccggg gccgccgcac ggcagcgtgc gngctgacac ctactgccgc ctctattcgc 1440
   tgagcgtgga caacttcaac gagntgctgg aggagtaccc catgatgcgg cgcgccttcg 1500
   agacggtggc catcgaccgc ctggaccgca tcggtgagcg ggccgggggc gtggccgggg 1560
   cgggtgccct ggcgggggag gggcgtggcc aaggcatcag gagagtggct tggacagtgg 1620
   cagggggaag ggcgtggctg tggcatcagg ggcacggttg gggcagagac gtggccaagg 1680
   catncaggag tgtggccatg gcagcagggg cgtggctggg gcaggggcag cggctggccg 1740
   ctcctaggac ccctttgggt ctagaggctg attttctgac ctattgtcct acttcagcca 1800
   gaggcagcct gtttcccaag ggagggaatg cacagggtgt ttgcggttgt gccgaatgct 1860
   cggtgagcac ctgctgtgtg ctgggggtgc aggggacaga cccgggggcc cactcagact 1920
   cccagggagg cttatggact ggtgatgaaa tcacacacga ctgggctgtg tgccagcagg 1980
   gcaggtgggg ccggtgggct tccctgagtt gggaatgcag agtggagacc agggtaaggg 2040
   atgccatgtg gaaacgggga ggaagatgtg ttcgtggagt ggacacagca catcccaagg 2100
   ccctgaggtg gaaaagaggc ctagagtcca gagagccagg gaggcctgga ggaggttggg 2160
   gaagaagggg aggccagaca cacagggccc agtgggcggc agggagagtt tagactaaat 2220
   caggagcatc agggagccat ggagggttct aggtgggcgg aggacctggt cagattgtat 2280
   ccgccaaggc gggccgtgtc caggagggag acggtgacct ggcctctcag gggggcagtc 2340
   tctggggcag ggagggncag agccctgatg actggatgta ggcgccagag agatggcggc 2400
   tcatgctgct gttcgtggga atgggaatga agaccatggc tgaaacgcag gacaggtgcg 2460
   acggagtggt gtcagggagc tccctggtgt acagtaggaa gctctccaca acttgctcta 2520
   tacagtgagt atgcaacccg ttcctgagta tcaggtgctt aggttataac ttctgtatac 2580
   agcaggtgct cagcacaggc tgtgtacagg caggtgtttt cggtatgcct gtggcacact 2640
   ggaggcagtc attacataat cagcgtatac aggtggtaca catgcatact tggtgcacag 2700
   tgatacctgc tccatgtaca cagcaggcat taaatacctg tttactgcca ggcgcggtgn 2760
   ntcacgcctg tagtcccagc actttcggag gccaaggtgg gtggatcacg aggtcaggag 2820
   attgagacca tcctggctaa catggtgaaa ccccgtctct actaaaaaaa aaaaaaaaaa 2880
   aaaaaa 2886
<210> 11
   <211> 2029
   <212> DNA
   <213> Homo sapiens
<400> 11
   gcgngccgcg tcgacgtggc ctccatgcca ctgtttgcca atgcggaccc caacttcgtg 60
   acgtccatgc tgaccaagct gcgttttgag gtcttccagc ctggggacta catcatccgg 120
   gaaggcacca ttggcaagaa gatgtacttc atccagcatg gcgtggtcag cgtgctcacc 180
   aagggcaaca aggagaccaa gctggccgac ggctcctact ttggagagat ctgcctgctg 240
   acccggggcc ggcgcacagc cagcgtgagg gccgacacct actgccgcct ctactcgctg 300
   agcgtggaca acttcaatga ggtgctggag gagtacccca tgatgcgaag ggccttcgag 360
   accgtggcgc tggaccgcct ggaccgcatt ggcaagaaga actccatcct cctccacaaa 420
   gtccagcacg acctcaactc cggcgtcttc aactaccagg agaatgagat catccagcag 480
   attgtgcagc atgaccggga gatggcccac tgcgcgcacc gcgtccaggc tgctgnctct 540
   gccaccccaa cccccacgcc cgtnatctgg accccgctga tccaggcacc actgcaggct 600
   gccgctgcca ccacttctgt ngccatagcc ctcacccacc acccytcgcn tgnctgytgc 660
   natnttnncg scctnccccc anggatctnn gggctggnca amctcggtgc cggnmagang 720
   ccaaggcacc tgnaacggct gnagtncctg atcccttctg cgctggtccg cctcgcccgc 780
   cagcagcccg tcccaggtgg acacaccgtc ttcatcctcc ttccacatcc aacagctggc 840
   tggattctct gcccccgctg gactgagccc actcctgccc tcatccagct cctccccacc 900
   ccccggggcc tgtggctccc cctcggctcc cacaccatca gctgcgtagc cgccaccacc 960
   atagccgggt ttggccactt ccacaaggcg ctgggtggct ccctgtcctc ctccgactct 1020
   cccctgctca ccccgctgca gccaggcgcc cgctccccgc aggctgccca gccatctccc 1080
   gcgccacccg gggcccgggg aggcctggga ctcccggagc acttcctgcc acccccaccc 1140
   tcatccagat ccccgtcatc tagccccggg cagctgggcc agcctcccgg ggagttgtcc 1200
   ctaggtctgg ccactggccc actgagcacg ccagagacac ccccacggca gcctgagccg 1260
   ccgtcccttg tggcaggggc ctctgggggn ggnttcccst gtaggncttt actccccgag 1320
   gaggtntcag cccccstggn ccacagccna gscccccnaa gaaccttccc gagtgccccg 1380
   ccccggncnt ctggctccca crgantcnnn cttryycctg ccacctgcat ccagcccccc 1440
   accaccccag ntcccccagc gccgggncac acccccgctc acccccggcc gcctcaccca 1500
   ggacctcaag ctcatctccg cgtctcagcc agccctgcct caggacgggg cgcagactct 1560
   ccgcagagcc tccccgcact cctcagggga gtccatggct gccttcccgc tcttccccag 1620
   ggctgggggt ggcagcgggg gcagtgggag cagcgggggc ctcggtcccc ctgggaggcc 1680
   ctatggtgcc atccccggcc agcacgtcac tctgcctcgg aagacatcct caggttcttt 1740
   gccaccccct ctgtctttgt ttggggcaag agccacctct tctggggggc cccctctgac 1800
   tgctggaccc cagagggaac ctggggccag gcctgagcca gtgcgctcca aactgccgtc 1860
   caatctatga gctgggccct tccttccctc ttctttcttc ttttctctcc cttccttctt 1920
   ccttcaggtt taactgtgat taggagatat accaataaca gtaataatta tttaaaaaac 1980
   cancasacac cagaaaaaca aaagacrrnc agaaagtcga cgcggccgc 2029
<210> 12
   <211> 2984
   <212> DNA
   <213> Bos taurus
<400> 12
   gggcaccagc cgcgccggag cccggagcgc agccactgag ggcagcggcg gcggcgggag 60
   cgaggcgcgc agcgagaagc ggcgcgggcg ggaagcagaa gccgccgccg ccgccgccgc 120
   cgccgcgacg ggcagccggg ctcggcggcc gccggatcgg gcccctgccc cctccgcctc 180
   gtgtccccgg cgccgggcgg ccggcgagtc tggagcccgc gccgtcgccg gccgcgtccc 240
   ccgggcatgg aaggaggcgg caagcccaac tcctcgtcca acagccggga cgatggcaac 300
   agcgtcttcc ccaccaaggc gcccgcgacg ggcgcggggc cggccgcggc cgagaagcgc 360
   ctgggcaccc cgccgggggg cggcgggacc ggcgcgaagg agcacggcaa ctcagtgtgc 420
   ttcaaggtgg acggcggcgg cggcggcggc gaggaatcgg ccgggggctt cgaggacgcc 480
   gaggggcccc ggcggcagta cggcttcatg cagcggcagt tcacctccat gctgcagccc 540
   ggggtcaaca aattctccct ccgcatgttc gggagccaga aggcggtgga gaaggagcag 600
   gaaagggtta aaactgcagg cttctggatt atccaccctt acagtgattt caggttttat 660
   tgggatttaa taatgcttat aatgatggtt ggaaatctgg tcatcatacc agttggaatc 720
   acattcttta cagaacagac aacaacacca tggattattt tcaatgtggc ttcagataca 780
   gttttccttt tggacttgat catgaatttc aggactggga ctgtcaatga agacagttct 840
   gaaatcatcc tggaccctaa agtgatcaag atgaattatt taaaaagctg gtttgtggtt 900
   gacttcatct catcaatccc agtggattat atctttctca ttgtagaaaa aggaatggat 960
   tcggaagttt acaagacagc cagggcactt cgcattgtga ggtttacaaa aattctcagt 1020
   ctcttgcgtt tattacgact ttcaaggtta attagataca tacatcagtg ggaagagatt 1080
   ttccacatga catatgatct tgccagtgct gtggtgagaa tttttaacct cattggcatg 1140
   atgctgctcc tgtgccactg ggatggctgt cttcagttcc tggtaccact gctgcaggac 1200
   ttcccaccag attgctgggt gtctctaaat gagatggtta atgattcttg gggaaagcag 1260
   tattcctacg cgctcttcaa agcgatgagt catatgctgt gcattggcta cggagcccaa 1320
   gcccccgtga gcatgtctga cctgtggatc accatgctga gcatgatcgt cggggccacc 1380
   tgctacgcca tgtttgttgg ccacgccacg gctctaattc agtctttgga ttcctcaagg 1440
   cggcaatatc aagagaagta taagcaagtg gaacaataca tgtcattcca taagttacca 1500
   gctgatatgc gtcagaagat acatgattat tatgaacaca gataccaagg caaaatcttt 1560
   gatgaggaaa atattctcaa tgaactcaat gatcctctga gagaggagat agtcaacttc 1620
   aactgccgaa aactagtggc tacaatgcct ctttttgcta atgcggatcc taatttcgtg 1680
   accgccatgc tgagcaagtt gagatttgag gtgtttcaac ctggagatta tatcatacga 1740
   gaaggagctg tgggtaaaaa aatgtatttc attcaacatg gtgttgctgg tgtcatcaca 1800
   aaatccagta aagaaatgaa gctgacagat ggctcatact ttggagagat ttgcttgctg 1860
   accaagggac ggcgcactgc cagtgttcga gctgatacat attgtcgtct ttactcactt 1920
   tctgtggaca atttcaatga ggtcctggag gaatatccaa tgatgagaag agcctttgag 1980
   acggttgcca ttgaccgatt agataggata gggaagaaaa attcaattct cctgcaaaag 2040
   ttccagaagg atctgaacac gggtgttttc aacaatcagg agaacgagat cctgaagcag 2100
   attgtgaaac acgacaggga aatggtgcag gcaatccctc ccctcaatta ccctcaaatg 2160
   acagccctga attccacctc ttcaactact accccgacct ctcgcctgag gacacagtca 2220
   ccgccagtgt acacagccac cagtctgtct catagcaacc tgcactcccc cagccccagc 2280
   acccagaccc cccagccgtc agccatcctc tcgccctgct cctacaccac cgctgtctgc 2340
   agccctcctg tacagagccc gctagccact cgaactttcc actatgcctc ccccacggct 2400
   tcccagttgt ccctcattca gcagcagcag gttcagcagc caccgcagcc ccagcagcca 2460
   ccccaacctc cacagacccc cggcagctcc acaccgaaaa acgaagtgca caagagcacg 2520
   caggcgcttc acaacaccag cctgacccga gaagtcaggc ccctctcggc ctcgcagccc 2580
   tcgctgcccc acgaggtctc caccctgatc tccagaccgc atcccactgt gggcgagtcc 2640
   ctggcctcca tccctcaacc cgtgaccacg gtccacggct cgggcctgca ggcagggggc 2700
   aggggcaccg tcccccagcg agtcaccctg ttccgacaga tgtcatcggg agccatcccc 2760
   cccaatcgag gagtcccccc ggccccccct ccaccagcag ccgctcatcc gagggaggcg 2820
   ccctcagtct taactacaga ctcagaggca gaaaagccac gatttgcttc aaatttatga 2880
   tcctgctgat tgtaaagcag aaagaaatac tctaacgtaa ctgaggacgc ttctcagatt 2940
   tgattttatt ctatctcctg atagatcctc tagcctacta tgaa 2984
<210> 13
   <211> 794
   <212> DNA
   <213> Rattus rattus
<400> 13
   tgcctgcagt tcctggtgcc catgctgcaa gacttcccca gcgactgctg gtgtccatca 60
   acaacatggt gaaccactcg tggagcgaac tctattcgtt cgcgctcttc aaggccatga 120
   gccacatgct ctgtattggc tacgggcggc aggctcccga gagcatgacg gacatctggc 180
   tcaccatgct cagcatgatc gtgggcgcca cctgctacgc tatgttcatt gggcacgcca 240
   cggcgcttat ccagtccctg gactcgtcac ggcgccagta ccaggagaag tacaagcaag 300
   tggagcagta catgtccttc cacaaactgc cggctgactt ccgccagaag atccacgatt 360
   actatgaaca ccggtaccag gggaagatgt ttgacgagga cagcatcctg ggggaactca 420
   acggcccact gcgtgaggag attgtgaact tcaactgccg gaagctggtg gcttccatgc 480
   cgttgtttgc caacgcagac cccaacttcg tcaccgccat gctgacaaag ctcaaatttg 540
   aggtcttcca gcctggagac tacatcatcc gagaggggac catcgggaag aagatgtact 600
   tcatccagca cggggtggtg agcgtgctca ccaagggcaa caaggagatg aagctgtcag 660
   atggctccta ttttggggag atctgcctgc tcacgagggg ccggcgcaca gccagtgtgc 720
   gggctgacac ctactgtcgc ctctactcac tgagcgtgga caacttcaac gaggtgctgg 780
   aggagtaccc catg 794
<210> 14
   <211> 649
   <212> DNA
   <213> Rattus rattus
<400> 14
   tccagcatgg gctgctcagt gtgttggcac ggggcgctcg ggacactcgc ctcactgacg 60
   gatcctactt tggggagatc tgcttgctga ctcgaggtcg gagaacagcc agtgtaaggg 120
   ctgacaccta ctgtcgcctc tactcactca gcgtggacca cttcaatgca gtgcttgagg 180
   agctcccgat gatgcgcagg gcttttgaga ctgtggccat ggaccggctt cggcgcatcg 240
   gcaaaaagaa ttcgatattg cagcggaaac gctctgagcc gagtccaggc agcagcagtc 300
   gtggcgtcat ggagcagcat ttggtacaac acgacagaga catggctcgt ggtattcggg 360
   gtctggctcc gggcacagga gcccgcctca gtggaaagcc agttctgtgg gaaccactgg 420
   tacacgcacc tcttcaggca gctgctgtga cctccaacgt ggccatagcc ttgactcatc 480
   agcgaggccc tctgcccctc tcccctgatt ctccagccac cctcctggct cgatctgcta 540
   gacgctcagc aggctcccca gcctccccac tggtgcctgt tcgagcaggt cctctgctgg 600
   cccggggacc ctgggcgtcc acttctcatc ttcctgccca cgggccctc 649
<210> 15
   <211> 4751
   <212> DNA
   <213> Homo sapiens
<400> 15
   tcgacaaaaa tgccagggaa aggcgagccc agagcttggt gatggagaaa ttgggaagcc 60
   accccccacc cttcaatctt aggatgggga attcgcaact gaagccggag cttcagactt 120
   ggggcgcact cccagcttag cccaggaaag agatttaagg gcgcagcagt gtggatacct 180
   ctcaccccgg ccccgaaggt ctagcgaggg tctaacctgg gccccttgcc aggcccgccc 240
   cccgcccctt tccagccccc ggcccgtgcg ccgctgcccc tttaagaagc ccaggtaggc 300
   aggcccggct gctggagccg ctcctatggc aacccgcgag ctgcggcggc ttcatgaata 360
   ttccggggcg cgggagcccg agcgctgccg gagggcgctt cgggggaggc ggccgctgat 420
   gtaagcccgg cgggtcgctg ggctccgctc ggttgcggcg ggagccccgg gacgggccgg 480
   acgggccggg gcagaggagg cgaggcgagc tcgcgggtgg ccagccacaa agcccgggcg 540
   gcgagacaga cggacagcca gccctcccgc gggacgcacg cccgggaccc gcgcgggccg 600
   tgcgctctgc actccggagc ggttccctga gcgccgcggc cgcagagcct ctccggccgg 660
   cgcccattgt tccccgcggg ggcggggcgc ctggagccgg gcggcgcgcc gcccctgaac 720
   gccagaggga gggagggagg caagaaggga gcgcggggtc cccgcgccca gccgggcccg 780
   ggaggaggtg tagcgcggcg agcccgggga ctcggagcgg gactaggatc ctccccgcgg 840
   cgcgcagcct gcccaagcat gggcgcctga ggctgccccc acgccggcgg caaaggacgc 900
   gtccccacgg gcggactgac cggcgggcgg acctggagcc cgtccgcggc gccgcgctcc 960
   tgcccccggc ccggtccgac cccggcccct ggcgccatgg acaagctgcc gccgtccatg 1020
   cgcaagcggc tctacagcct cccgcagcag gtgggggcca aggcgtggat catggacgag 1080
   gaagaggacg ccgaggagga gggggccggg ggccgccaag accccagccg caggagcatc 1140
   cggctgcggc cactgccctc gccctccccc tcggcggccg cgggtggcac ggagtcccgg 1200
   agctcggccc tcggggcagc ggacagcgaa gggccggccc gcggcgcggg caagtccagc 1260
   acgaacggcg actgcaggcg cttccgcggg agcctggcct cgctgggcag ccggggcggc 1320
   ggcacgggcg gcacggggag cggcagcagt cacggacacc tgcatgactc cgcggaggag 1380
   cggcggctca tcgccgaggg cgacgcgtcc cccggcgagg acaggacgcc cccaggcctg 1440
   gcggccgagc ccgagcgccc cggcgcctcg gcgcagcccg cagcctcgcc gccgccgccc 1500
   cagcagccac cgcagccggc ctccgcctcc tgcgagcagc cctcggtgga caccgctatc 1560
   aaagtggagg gaggcgcggc tgccggcgac cagatcctcc cggaggccga ggtgcgcctg 1620
   ggccaggccg gcttcatgca gcgccagttc ggggccatgc tccaacccgg ggtcaacaaa 1680
   ttctccctaa ggatgttcgg cagccagaaa gccgtggagc gcgaacagga gagggtcaag 1740
   tcggccggat tttggattat ccacccctac agtgacttca gattttactg ggacctgacc 1800
   atgctgctgc tgatggtggg aaacctgatt atcattcctg tgggcatcac cttcttcaag 1860
   gatgagaaca ccacaccctg gattgtcttc aatgtggtgt cagacacatt cttcctcatc 1920
   gacttggtcc tcaacttccg cacagggatc gtggtggagg acaacacaga gatcatcctg 1980
   gacccgcagc ggattaaaat gaagtacctg aaaagctggt tcatggtaga tttcatttcc 2040
   tccatccccg tggactacat cttcctcatt gtggagacac gcatcgactc ggaggtctac 2100
   aagactgccc gggccctgcg cattgtccgc ttcacgaaga tcctcagcct cttacgcctg 2160
   ttacgcctct cccgcctcat tcgatatatt caccagtggg aagagatctt ccacatgacc 2220
   tacgacctgg ccagcgccgt ggtgcgcatc gtgaacctca tcggcatgat gctcctgctc 2280
   tgccactggg acggctgcct gcagttcctg gtacccatgc tacaggactt ccctgacgac 2340
   tgctgggtgt ccatcaacaa catggtgaac aactcctggg ggaagcagta ctcctacgcg 2400
   ctcttcaagg ccatgagcca catgctgtgc atcggctacg ggcggcaggc gcccgtgggc 2460
   atgtccgacg tctggctcac catgctcagc atgatcgtgg gtgccacctg ctacgccatg 2520
   ttcattggcc acgccactgc cctcatccag tccctggact cctcccggcg ccagtaccag 2580
   gaaaagtaca agcaggtgga gcagtacatg tcctttcaca agctcccgcc cgacacccgg 2640
   cagcgcatcc acgactacta cgagcaccgc taccagggca agatgttcga cgaggagagc 2700
   atcctgggcg agctaagcga gcccctgcgg gaggagatca tcaactttaa ctgtcggaag 2760
   ctggtggcct ccatgccact gtttgccaat gcggacccca acttcgtgac gtccatgctg 2820
   accaagctgc gtttcgaggt cttccagcct ggggactaca tcatccggga aggcaccatt 2880
   ggcaagaaga tgtacttcat ccagcatggc gtggtcagcg tgctcaccaa gggcaacaag 2940
   gagaccaagc tggccgacgg ctcctacttt ggagagatct gcctgctgac ccggggccgg 3000
   cgcacagcca gcgtgagggc cgacacctac tgccgcctct actcgctgag cgtggacaac 3060
   ttcaatgagg tgctggagga gtaccccatg atgcgaaggg ccttcgagac cgtggcgctg 3120
   gaccgcctgg accgcattgg caagaagaac tccatcctcc tccacaaagt ccagcacgac 3180
   ctcaactccg gcgtcttcaa ctaccaggag aatgagatca tccagcagat tgtgcagcat 3240
   gaccgggaga tggcccactg cgcgcaccgc gtccaggctg ctgcctctgc caccccaacc 3300
   cccacgcccg tcatctggac cccgctgatc caggcaccac tgcaggctgc cgctgccacc 3360
   acttctgtgg ccatagccct cacccaccac cctcgcctgc ctgctgccat cttccgccct 3420
   cccccaggat ctgggctggg caacctcggt gccgggcaga cgccaaggca cctgaaacgg 3480
   ctgcagtccc tgatcccttc tgcgctgggc tccgcctcgc ccgccagcag cccgtcccag 3540
   gtggacacac catcttcatc ctccttccac atccaacagc tggctggatt ctctgccccc 3600
   gctggactga gcccactcct gccctcatcc agctcctccc caccccccgg ggcctgtggc 3660
   tccccctcgg ctcccacacc atcagctggc gtagccgcca ccaccatagc cgggtttggc 3720
   cacttccaca aggcgctggg tggctccctg tcctcctccg actctcccct gctcaccccg 3780
   ctgcagccag gcgcccgctc cccgcaggct gcccagccat ctcccgcgcc acccggggcc 3840
   cggggaggcc tgggactccc ggagcacttc ctgccacccc caccctcatc cagatccccg 3900
   tcatctagcc ccgggcagct gggccagcct cccggggagt tgtccctagg tctggccact 3960
   ggcccactga gcacgccaga gacaccccca cggcagcctg agccgccgtc ccttgtggca 4020
   ggggcctctg ggggggcttc ccctgtaggc tttactcccc gaggaggtct cagcccccct 4080
   ggccacagcc caggcccccc aagaaccttc ccgagtgccc cgccccgggc ctctggctcc 4140
   cacggatcct tgctcctgcc acctgcatcc agccccccac caccccaggt cccccagcgc 4200
   cggggcacac ccccgctcac ccccggccgc ctcacccagg acctcaagct catctccgcg 4260
   tctcagccag ccctgcctca ggacggggcg cagactctcc gcagagcctc cccgcactcc 4320
   tcaggggagt ccatggctgc cttcccgctc ttccccaggg ctgggggtgg cagcgggggc 4380
   agtgggagca gcgggggcct cggtccccct gggaggccct atggtgccat ccccggccag 4440
   cacgtcactc tgcctcggaa gacatcctca ggttctttgc caccccctct gtctttgttt 4500
   ggggcaagag ccacctcttc tgggggcccc cctctgactg ctggacccca gagggaacct 4560
   ggggccaggc ctgagccagt gcgctccaaa ctgccgtcca atctatgagc tgggcccttc 4620
   cttccctctt ctttcttctt ttctctccct tccttcttcc ttcaggttta actgtgatta 4680
   ggagatatac caataacagt aataattatt taaaaaacca cacacaccag aaaaacaaaa 4740
   gacagcagaa a                                                      4751
<210> 16
   <211> 29
   <212> DNA
   <213> Künstliche Sequenz
<220>
   <223> Degenerierter Primer

<400> 16
   ctgactgcag argtnttyca rccnggnga 29

<210> 17
   <211> 28
   <212> DNA
   <213> Künstliche Sequenz
<220>
   <223> Degenerierter Primer

<400> 17
   atcggaattc nccraartan ganccrtc 28

## Patentansprüche

1. Nukleinsäure, vorzugsweise DNA, die für einen humanen Iₕ-Ionenkanal kodiert, **dadurch gekennzeichnet, dass** sie die Sequenz gemäß SEQ ID NO: 15 umfasst.

2. Nukleinsäure, vorzugsweise DNA, **dadurch gekennzeichnet, dass** deren Sequenz mindestens 80% Identität zu einer Nukleinsäure mit SEQ ID NO: 15 aufweist und für einen humanen Iₕ-Ionenkanal kodiert, wobei die Identität über die vollständigen Sequenzen berechnet wird.

3. Nukleinsäure nach Anspruch 2, **dadurch gekennzeichnet, dass** die Identität mindestens 90% beträgt, wobei die Identität über die vollständigen Sequenzen berechnet wird.

4. Nukleinsäuresequenz, umfassend eine komplementäre Sequenz zur Nukleinsäuresequenz nach Anspruch 3.

5. Nukleinsäure nach einem der Ansprüche 1 bis 4, **dadurch gekennzeichnet, dass** sie eine RNA ist.

6. Ein humanes Iₕ-Ionenkanal-Polypeptid, kodiert von einer Nukleinsäure nach einem der Ansprüche 1 bis 3, oder 5, wobei der Iₕ-Ionenkanal kodiert wird von der Nukleinsäure mit SEQ ID NO: 15 oder einer Nukleinsäure mit 80% Identität zur SEQ ID NO: 15, wobei die Identität über die vollständigen Sequenzen berechnet wird.

7. Verwendung einer Nukleinsäure nach einem der Ansprüche 1 bis 5 zum Identifizieren von Substanzen, die die Aktivität des Ionenkanals beeinflussen.

8. Nukleinsäure nach einem der Ansprüche 1 bis 5 zur Verwendung in der Therapie und/oder Diagnose.

9. Verfahren zum Identifizieren von Substanzen mit Wirkung auf Ionenkanäle, umfassend den Schritt des Inkontaktbringens der zu testenden Substanz mit einem Nukleinsäuremolekül nach einem der Ansprüche 1 bis 5 und/oder einem Polypeptid nach Anspruch 6.

10. Verfahren nach Anspruch 9, umfassend die folgenden Schritte:
- Erzeugung homogener Kanalpräparate mit Hilfe des Nukleinsäuremoleküls gemäß einem der Ansprüche 1 bis 5 und/oder einem Polypeptid nach Anspruch 6.
- Testen von Substanzen an dieser Kanalpräparation.

11. Kit zur Durchführung eines Verfahrens nach Anspruch 9 und/oder 10, enthaltend mindestens eine Nukleinsäure gemäß einem oder mehreren der Ansprüche 1 bis 5 und/oder ein Polypeptid nach Anspruch 6.

12. *In vitro*-Verfahren für die Erkennung einer Herz-/Kreislaufstörung, **dadurch gekennzeichnet, dass** eine Nukleinsäure nach einem der Ansprüche 1 bis 5 mit Patientenmaterial in Kontakt gebracht wird, umfassend den folgenden Schritt:
- Inkontaktbringen der Nukleinsäure des Patienten mit einer Nukleinsäure gemäß einem der Ansprüche 1 bis 5, so dass ein Signal erhalten wird, das die Anwesenheit und/oder die Abwesenheit einer Ionenkanalsequenz anzeigt.

13. Verwendung einer oder mehrerer Nukleinsäuren nach einem der Ansprüche 1 bis 5 und/oder eines Polypeptids nach Anspruch 6 für die Herstellung eines Medikaments zur Behandlung und/oder Prophylaxe von Herz-/Kreislaufstörungen, Bewusstseinsstörungen und/oder Schmerzzuständen.

14. Verwendung nach Anspruch 13, **dadurch gekennzeichnet, dass** die Herz/Kreislauf-Störungen auf einer Fehlsteuerung des Sinusknotens beruhen.

15. Verwendung nach Anspruch 13, **dadurch gekennzeichnet, dass** die Bewusstseinsstörungen auf einer Fehlfunktion in thalamischen Neuronen beruhen.

16. Pharmazeutische Zusammensetzung, umfassend eine oder mehrere der Nukleinsäuren nach einem der Ansprüche 1 bis 5 und/oder ein Polypeptid nach Anspruch 6.

17. Konstrukt, enthaltend eine Nukleinsäure nach einem der Ansprüche 1 bis 5.

18. Isolierte Wirtszelle, enthaltend ein Konstrukt nach Anspruch 17.

## Claims

1. Nucleic acid, preferably DNA, which codes for a human Iₕ ion channel, **characterized in that** it comprises the sequence according to SEQ ID NO: 15.

2. Nucleic acid, preferably DNA, **characterized in that** the sequence thereof exhibits at least 80% identity to a nucleic acid with sequence SEQ ID NO: 15 and codes for a human Iₕ ion channel, the identity being calculated through the complete sequences.

3. Nucleic acid according to claim 2, **characterized in that** the identity is at least 90%, the identity being calculated through the complete sequences.

4. Nucleic acid sequence comprising a complementary sequence to the nucleic acid sequence according to claim 3.

5. Nucleic acid according to one of claims 1 to 4, **characterized in that** it is an RNA.

6. A human Iₕ ion channel polypeptide encoded by a nucleic acid according to one of claims 1 to 3 or 5, wherein the Iₕ ion channel is encoded by the nucleic acid with SEQ ID NO: 15 or a nucleic acid with 80% identity to SEQ ID NO: 15, the identity being calculated through the complete sequences.

7. Use of a nucleic acid according to one of claims 1 to 5 for identifying substances which influence the activity of the ion channel.

8. Nucleic acid according to one of claims 1 to 5 for use in therapy and/or diagnosis.

9. Method for identifying substances with effect on ion channels, comprising the step of contacting the substance to be tested with a nucleic acid molecule according to one of claims 1 to 5 and/or a polypeptide according to claim 6.

10. Method according to claim 9, comprising the following steps:
- producing homogeneous channel preparations with the help of the nucleic acid molecule according to one of claims 1 to 5 and/or a polypeptide according to claim 6,
- testing substances on said channel preparation.

11. Kit for carrying out a method according to claim 9 and/or 10, comprising at least one nucleic acid according to one or several of claims 1 to 5 and/or a polypeptide according to claim 6.

12. *In-vitro* method for detecting a cardiovascular disorder, **characterized in that** a nucleic acid according to one of claims 1 to 5 is contacted with material from a patient, comprising the following step:
- contacting the nucleic acid from the patient with a nucleic acid according to one of claims 1 to 5, whereby a signal is obtained that is indicative of the presence and/or absence of an ion channel sequence.

13. Use of one or more nucleic acids according to one of claims 1 to 5 and/or of a polypeptide according to claim 6 for preparing a medicament for the treatment and/or prophylaxis of cardiovascular disorders, disturbances of consciousness and/or pain states.

14. Use according to claim 13, **characterized in that** the cardiovascular disorders are due to a faulty control of the sinus node.

15. Use according to claim 13, **characterized in that** the disturbances of consciousness are due to a faulty function in thalamic neurons.

16. Pharmaceutical composition comprising one or more of the nucleic acids according to one of claims 1 to 5 and/or a polypeptide according to claim 6.

17. Construct containing a nucleic acid according to one of claims 1 to 5.

18. Isolated host cell containing a construct according to claim 17.

## Revendications

1. Acide nucléique, de préférence ADN, codant pour un canal ionique Iₕ humain, **caractérisé en ce qu'**il comprend la séquence selon SEQ ID NO : 15.

2. Acide nucléique, de préférence ADN, **caractérisé en ce que** sa séquence présente une identité d'au moins 80% avec un acide nucléique ayant la séquence SEQ ID NO : 15, et code pour un canal ionique Iₕ humain, l'identité étant calculée par rapport aux séquences complètes.

3. Acide nucléique selon la revendication 2, **caractérisé en ce que** l'identité est d'au moins 90 %, l'identité étant calculée par rapport aux séquences complètes.

4. Séquence d'acide nucléique, comportant une séquence complémentaire de la séquence d'acide nucléique selon la revendication 3.

5. Acide nucléique selon l'une des revendications 1 à 4, **caractérisé en ce qu'**il s'agit d'un ARN.

6. Polypeptide-canal ionique Iₕ humain, codé par un acide nucléique selon l'une des revendications 1 à 3, ou selon la revendication 5, le canal ionique Iₕ étant codé par l'acide nucléique ayant la séquence SEQ ID NO : 15 ou par un acide nucléique présentant une identité de 80 % avec la séquence SEQ ID NO : 15, l'identité étant calculée par rapport aux séquences complètes.

7. Utilisation d'un acide nucléique selon l'une des revendications 1 à 5 pour identifier des substances qui influent sur l'activité du canal ionique.

8. Acide nucléique selon l'une des revendications 1 à 5, pour utilisation en thérapie et/ou diagnostic.

9. Procédé d'identification de substances ayant un effet sur les canaux ioniques, comprenant l'étape de mise en contact de la substance à tester, avec une molécule d'acide nucléique selon l'une des revendications 1 à 5 et/ou un polypeptide selon la revendication 6.

10. Procédé selon la revendication 9, comprenant les étapes suivantes:
- production de préparations homogènes de canal à l'aide de la molécule d'acide nucléique selon l'une des revendications 1 à 5 et/ou d'un polypeptide selon la revendication 6,
- test de substances sur cette préparation de canal.

11. Kit pour la mise en oeuvre d'un procédé selon la revendication 9 et/ou 10, contenant au moins un acide nucléique selon une ou plusieurs des revendications 1 à 5 et/ou un polypeptide selon la revendication 6.

12. Procédé *in vitro* pour la mise en évidence d'un trouble cardiovasculaire, **caractérisé en ce qu'**on met en contact un acide nucléique selon l'une des revendications 1 à 5 avec une substance provenant d'un patient, comprenant l'étape suivante:
- mise en contact de l'acide nucléique du patient avec un acide nucléique selon l'une des revendications 1 à 5 de façon à obtenir un signal qui indique la présence et/ou l'absence d'une séquence de canal ionique.

13. Utilisation d'un ou de plusieurs acides nucléiques selon l'une des revendications 1 à 5 et/ou d'un polypeptide selon la revendication 6 pour la préparation d'un médicament destiné au traitement et/ou à la prophylaxie de troubles cardiovasculaires, de troubles de la conscience et/ou d'états douloureux.

14. Utilisation selon la revendication 13, **caractérisée en ce que** les troubles cardiovasculaires proviennent d'un défaut de commande du noeud sinusal.

15. Utilisation selon la revendication 13, **caractérisée en ce que** les troubles de la conscience proviennent d'un dysfonctionnement dans les neurones thalamiques.

16. Composition pharmaceutique comprenant un ou plusieurs des acides nucléiques selon l'une des revendications 1 à 5 et/ou un polypeptide selon la revendication 6.

17. Produit d'assemblage contenant un acide nucléique selon l'une des revendications 1 à 5.

18. Cellule hôte isolée contenant un produit d'assemblage selon la revendication 17.
